(19) Europäisches Patentamt
European Patent Office
Office européen des brevets

(11) **EP 1 890 762 B1**

(12) **EUROPEAN PATENT SPECIFICATION**

(45) Date of publication and mention
of the grant of the patent:
**02.12.2020 Bulletin 2020/49**

(51) Int Cl.:
**A61N 2/02** *(2006.01)*     **A61N 2/00** *(2006.01)*

(21) Application number: **06756220.7**

(22) Date of filing: **15.06.2006**

(86) International application number:
**PCT/IL2006/000694**

(87) International publication number:
**WO 2006/134598 (21.12.2006 Gazette 2006/51)**

(54) **TRANSCRANIAL MAGNETIC STIMULATION SYSTEM**

TRANSKRANIELLES MAGNETISCHES STIMULATIONSSYSTEM

SYSTEME DE STIMULATION MAGNETIQUE TRANSCRANIENNE

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.06.2005 US 153905**

(43) Date of publication of application:
**27.02.2008 Bulletin 2008/09**

(73) Proprietors:
• **Brainsway, Inc.**
  **Wilmington, DE 19801 (US)**
• **The Government of the United States**
  **As represented by the Secretary of Health & Human**
  **Services,**
  **Bethesda, MD 20892 (US)**
• **Yeda Research and Development Co. Ltd.**
  **76100 Rehovot (IL)**

(72) Inventors:
• **ZANGEN, Abraham**
  **60000 Jerusalem (IL)**

• **ROTH, Yiftach**
  **60000 Rechelim (IL)**
• **MIRANDA, Pedro C.**
  **P-1600-581 Lisbon (PT)**
• **HAZANI, David**
  **60000 Rechelim (IL)**
• **HALLET, Mark**
  **Bethesda, Maryland 20816 (US)**

(74) Representative: **HGF**
**1 City Walk**
**Leeds LS11 9DX (GB)**

(56) References cited:
**EP-A1- 0 692 993        WO-A2-02/32504**
**WO-A2-02/32504        GB-A- 2 196 853**
**JP-A- H08 280 820      US-A- 5 738 625**
**US-A1- 2004 199 041    US-B1- 6 179 771**
**US-B1- 6 537 197**

EP 1 890 762 B1

**Description**

**FIELD OF THE INVENTION**

The present invention relates to a system for deep transcranial magnetic stimulation, and more particularly, to an improved system for stimulating specific regions of the brain while minimizing pain and side effects.

**BACKGROUND OF THE INVENTION**

Transcranial magnetic stimulation (TMS) is widely used as a research tool to study aspects of the human brain and has been suggested as a diagnostic and therapeutic tool, especially in neurology and psychiatry.

Biological tissue can be stimulated using magnetic fields produced by passing electrical currents through electrically conductive materials positioned adjacent to the tissue. The magnetic fields are intended to induce an electric field in a tissue, provided that the tissue is a conductive medium. More specifically, magnetic stimulation can cause electric conduction in brain cells, and, as a consequence, generation of action potentials.

The magnetic stimulation is delivered or generated by a TMS system, including a stimulation circuit and a coil positioned on the patient's scalp, inducing nerve stimulation within the brain. The TMS stimulation circuit generally includes a high voltage power supply which charges a capacitor or bank of capacitors, which are then rapidly discharged via an electronic switch into the TMS coil to produce a briefly changing magnetic field pulse. A typical circuit includes a transformer, such as a high-voltage transformer, IGBT transistor, or other device used to transform low-voltage AC into high voltage DC. The switch, which may be, for example, a thyristor switch, must be able to traverse high current at short intervals (such as 50-250 [mu]s).

Current magnetic stimulation techniques and coils are suitable for superficial stimulation of the brain, whereas for some medical indications, deeper stimulation would be essential. For example, standard superficial stimulation does not induce effective stimulation directly in deep prefrontal cortex areas (3-4 cm deep) and other reward and mood-related brain structures such as the nucleus accumbens (ventral striatum). Direct stimulation of such deep regions may be more effective for the treatment of major depression than superficial cortical stimulation obtained with a standard TMS system. Stimulation of deep brain regions may be beneficial for many other deep-brain related disorders and other psychiatric and neurological disorders such as autism, post-traumatic stress disorder (PTSD), addictive behaviors including smoking, overeating and drug addiction, schizophrenia, Parkinson's disease, and others. Stimulation of deep brain regions requires a very high intensity which cannot be reached by the magnetic stimulators available today, using standard circular or figure-eight coils. Moreover, stimulators or coils which may provide greater electromagnetic fields, if used for deep-brain stimulation, may cause undesirable side effects, such as, for example, epileptic seizures or other problems associated with over-stimulation of cortical regions.

An attempt to solve this problem is disclosed, for example, in U.S. Patent Publication Number 2005/0228209. A method disclosed therein for TMS includes moving one or more coils to a target area, and applying magnetic fields to the target from multiple locations such that at any given time, the brain surface receives only a small amount of magnetic energy. However, the method disclosed therein does not address the physiological properties associated with stimulating action potentials in neuronal structures, and does not include any discussion of temporal control of the timing of impulses from different sources.

A novel approach to deep brain TMS with minimal surface stimulation has been previously described in International Publication Number WO 02/32504. The device described therein includes a base and an extension portion, the base having individual windings for individual paths of current flow, and the extension portion designed so as to minimize unwanted stimulation of other regions of the brain. US5,738,625 discloses a method of, and apparatus for, magnetically stimulating neural cells. GB2196853A discloses a tissue stimulator. JP408280820 discloses a magnetic stimulating device for a living body. EP0692993, upon which the preamble of claim 1 is based, discloses an apparatus for the magnetic stimulation of cells or tissue.

**SUMMARY OF THE INVENTION**

The invention is defined in the appended claims. There is provided a system for TMS which is capable of activating a neuronal structure.

According to another example of the present disclosure, there is provided a coil for magnetic stimulation of a target area. The coil is positionable on a body part, and includes a base portion having multiple spaced apart members for providing electrical current flow in a direction tangential to the preferred direction for activation of the target area, the base portion positioned at a first level with respect to the target area, and a contacting return portion for carrying returning current in a direction opposite the target area, the contacting return portion in electrical communication with at least one member and positioned substantially in the first level and spaced at a distance from the target area.

In some examples, the coil may also include a protruding return portion for carrying returning current in a direction opposite the preferred direction, the protruding return portion in electrical communication with at least one member and positioned at a second level with respect to the target area, the second level located at a distance above the first level. Some of the members may be in electrical communication with the protruding return portion and some of the members may be in electrical communication with the contacting return portion. In some examples, at least one member is positioned in a lateral- medial direction and in some embodiments, at least one member is positioned in an anterior-posterior direction. In some examples, the multiple members are substantially parallel to one another. In some examples, the first level is the skull and the second level is a distance above the skull. The distance may range from 4-10 cm and may be around 7 cm. A distance of the contacting return portion from the target area may be in a range of 7-10 cm. In some examples, the base portion has a substantially arch-like configuration which is complementary to the body part. In some embodiments, the body part is the head and the target area is a portion of a brain. The portion of the brain targeted may be at least 3 cm deep.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

BRIEF DESCRIPTION OF THE DRAWINGS

The above and further advantages of the present invention may be better understood by referring to the following description in conjunction with the accompanying drawings in which:

FIG. 1A is a schematic illustration of a system not falling within the scope of the present invention;

FIG. 1B is an illustration of a helmet from the system of FIG. 1A;

FIG. 2A is an illustration of a device including a frame and an electrically conductive coil having a base and an outwardly projecting extension portion;

FIG. 2B is a schematic diagram illustrating conducting wires and current flow in the embodiment illustrated in FIG. 2A;

FiG. 3A is an illustration of a device including a frame and an electrically conductive coil having a base and an outwardly projecting extension portion with a plurality of radially elongated extension elements;

FIG. 3B is a schematic diagram illustrating conducting wires and current flow in the embodiment illustrated in FIG. 3A;

FIG. 4 is an illustration of a coil for TMS;

FIG. 5 is an illustration of another coil for TMS;

FIG. 6 is a block diagram illustration of a cooling system;

FIG. 7 is a block diagram illustration of external cooling unit from the cooling system depicted in FIG.6;

FIG. 8 is a block diagram illustration of a liquid circulator from the cooling system depicted in FIG. 6;

FIG. 9 is a schematic illustration of an internal system in contact with coils illustrated in FIGS. 4 and 5;

FIG. 10 is a graphical illustration of a strength-duration curve reflecting the average of four subjects, using eight different coils with inductance L of between 6 and 148 [mu]H;

FIG. 11 is a graphical illustration depicting pulses produced by TMS coils having inductances of 13 and 70 [mu]H;

FIG. 12 is a block diagram illustration of a multi-channel TMS system in accordance with embodiments of the present invention;

FIG. 13 is a graphical illustration of electric field pulses induced in a deep brain region, a first cortical region, and a second cortical region when stimulated according to the present invention;

FIG. 14 is a graphical illustration of electric field pulses induced in a deep brain region and three cortical regions when stimulated according to parameters not falling within the scope of the present invention;

FIG. 15 is a graphical illustration of electric field pulses induced in a deep brain region and three cortical regions when stimulated according to parameters not falling within the scope of the present invention, with different parameters than those shown in FIG. 14;

FIG. 16 is an illustration of a coil designed to stimulate the right abductor pollicis brevis in an experimental trial on humans; and

FIG. 17 is a graphical illustration of the results of performing stimulation using the coil of FIG. 16 as compared to a standard figure-8 coil.

It will be appreciated that for simplicity and clarity of illustration, elements shown in the drawings have not necessarily been drawn accurately or to scale. For example, the dimensions of some of the elements may be exaggerated relative to other elements for clarity or several physical components may be included in one functional block or element. Further, where considered appropriate, reference numerals may be repeated among the drawings to indicate corresponding or analogous elements. Moreover, some of the blocks depicted in the drawings may be combined into a single function.

## DETAILED DESCRIPTION

In the following detailed description, numerous specific details are set forth in order to provide a thorough understanding of the present invention. It will be understood by those of ordinary skill in the art that the present invention may be practiced without these specific details. In other instances, well-known methods, procedures, components and structures may not have been described in detail so as not to obscure the present invention.

The present invention is a system for stimulating deep brain regions using TMS. Specifically, the present invention can be used to stimulate deep regions of the brain while maintaining a high percentage of field intensity as compared to superficial regions.

The principles and operation of a system for transcranial magnetic stimulation according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

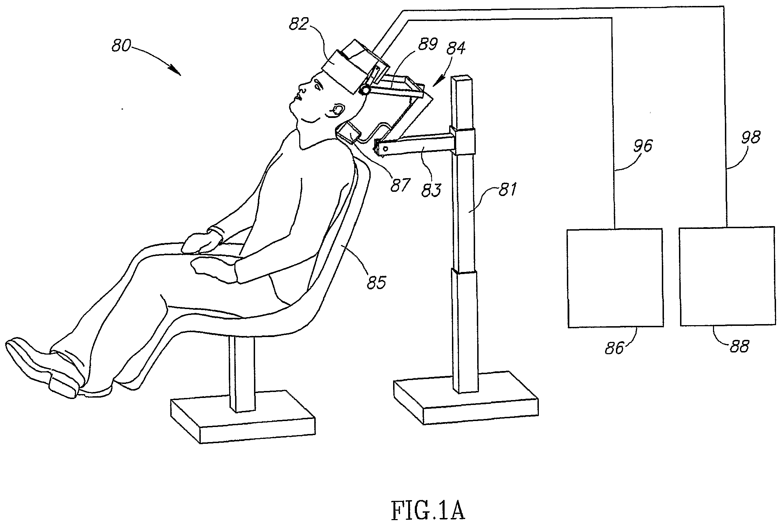

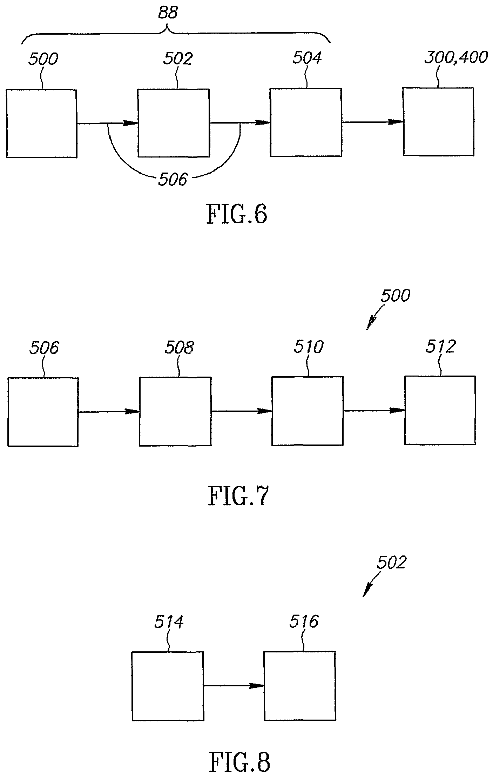

Reference is now made to FIG. 1A, which is a schematic illustration of a system 80 in accordance with an example present disclosure. System 80 includes a helmet 82 which holds coils for magnetic stimulation and is positionable around a head of a subject. Helmet 82 is adjustable via positioning portion 84. Positioning portion includes a stand 81 with an adjustable arm 83, a chair 85 with a rear head support 87, and an adaptor 89 between helmet 82 and adjustable arm 83. A stimulator 86 is in electrical communication with the coils of helmet 82, and is designed to provide electrical stimulation to the coils. Stimulator 86 is a commercially available neurostimulator, such as any of the various models of magnetic stimulators produced by Medtronic, Inc. of Minneapolis, MN, USA (e.g., MagPro, MagLite Compact), or power supplies sold with various models of magnetic stimulators produced by Magstim Company US, LLC, of New York, NY, USA (e.g., Magstim Model 200, Magstim Model 220, Magstim Model 250, BiStim, Magstim Rapid, Magstim QuadroPulse, Magstim Rapid<2>). Stimulator 86 is used to deliver electrical stimulation to the brain, and provides a controlled output, frequency, and pulse duration, and may also include an indication of coil temperature. A cooling system 88 is also in communication with the coils of helmet 82, and is designed to maintain an ambient temperature in the coils during repetitive stimulation provided by stimulator 86. Cooling system 88 may be a system based on air cooling using a Freon system, or a thermoelectric cooler (TEC) system such as the TECs produced by Melcor Ltd, (Trenton, NJ, USA), with either open air pathways or closed, two-direction air pathways, or cooling system 88 may be a liquid cooling system. A particular example of a cooling system 88, designed specifically for use with the coils of the present invention, will be described in further detail herein below with respect to FIGS. 6-9.

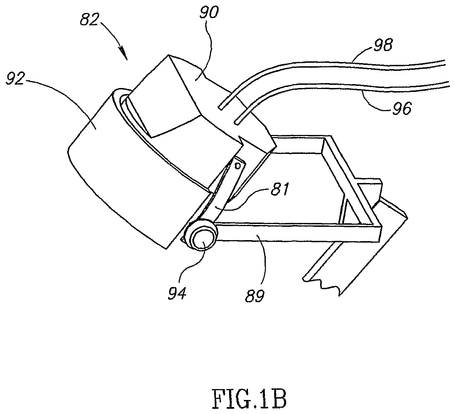

Reference is now made to FIG. 1B, which is a more detailed schematic illustration of helmet 82 in accordance with a preferred example. Helmet 82 includes a rigid cover 90 and a flexible cover 92. Flexible cover 92 is designed to provide flexibility over the head. An internal wall of helmet 82 is lined with a sponge for padding. In one embodiment, the liner is a 0.9 mm biocompatible sponge with one-sided glue (3M Foam Medical Tape). Adaptor 89 is attached to an upper portion of stand 81 via an adjustment screw 94. Adjustment screw 94 enables adjustment of the height or angle of helmet 82. Wires 96, 98 run from stimulator 86 and cooling system 88 to helmet 82.

Positioned within helmet 82 are coils for transcranial magnetic stimulation. Coils are designed to penetrate deep regions of the brain, while minimizing adverse side effects. The basic principles of operation of coils suitable for deep brain stimulation are as follows:

1. Proper orientation of stimulating coils.

Coils must be oriented such that they will produce a considerable field in a direction tangential to the surface, which should also be the preferable direction to activate the neurons under consideration. That is, wires of the coils are directed in one or more directions, which results in a preferred activation of neuronal structures orientated in these particular directions. In some cases, there is one preferred direction along the length or width axis, and in other cases, there are two or more preferred directions along both the length and width axes. Thus, the placement and orientation of activating coils on the skull is important.

2. Minimization of non-tangential coil elements.

Electrical field intensity in the tissue to be stimulated and the rate of decrease of electrical field as a function of distance from the coil depend on the orientation of the coil elements relative to the tissue surface. It has been shown that coil elements which are perpendicular to the surface induce accumulation of surface charge, which leads to cancellation of the perpendicular component of the induced field at all points within the tissue, and in general to reduction of the electrical field in all other directions in most points. Thus, the length of coil elements which are not tangential to the brain tissue surface should be minimized. Furthermore, the non-tangential coil elements should be as small as possible and placed as far as possible from the deep region to be activated. The combination of these two factors helps to minimize accumulation of surface charge.

3. Maximization of the field in the deep region as compared with the field at the cortex.

A major goal of deep TMS is to maximize deep region stimulation without causing a large electrical field at surface areas of the brain. If the electrical field at the surface areas is too large, it can cause pain, epileptic seizures, or other complications. Thus, it is important to try to maximize deep region stimulation without causing a large electrical field to accumulate at surface areas. This can be accomplished by summation of electrical impulses, a concept which will be described further hereinbelow. In addition the coil elements leading currents in a direction opposite to the preferred direction (the return paths), should be located far from the desired brain region.

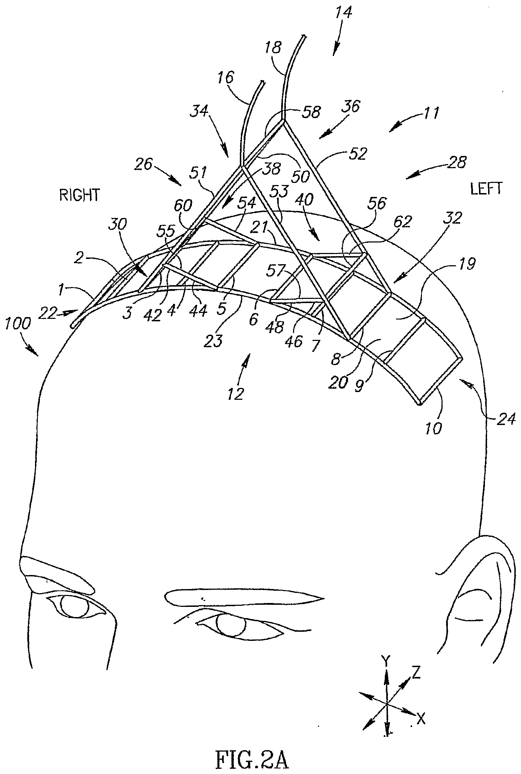

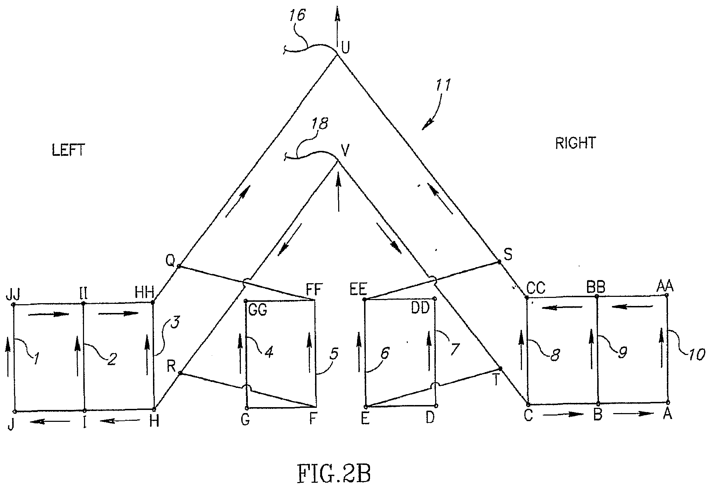

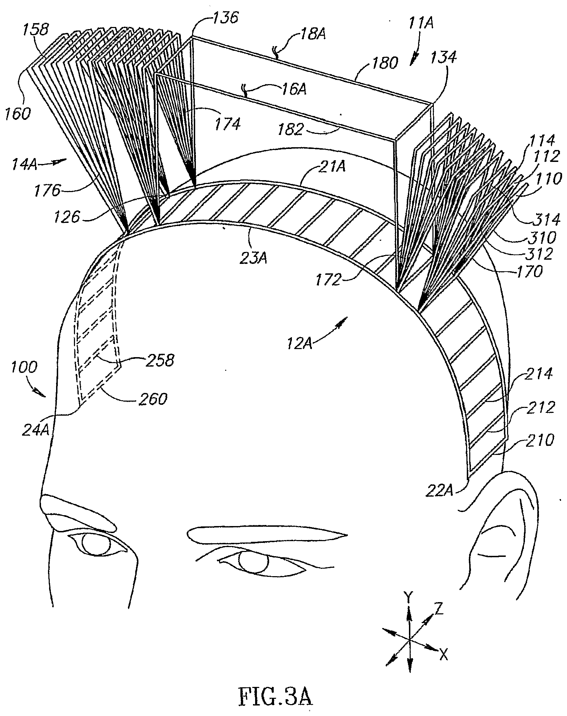

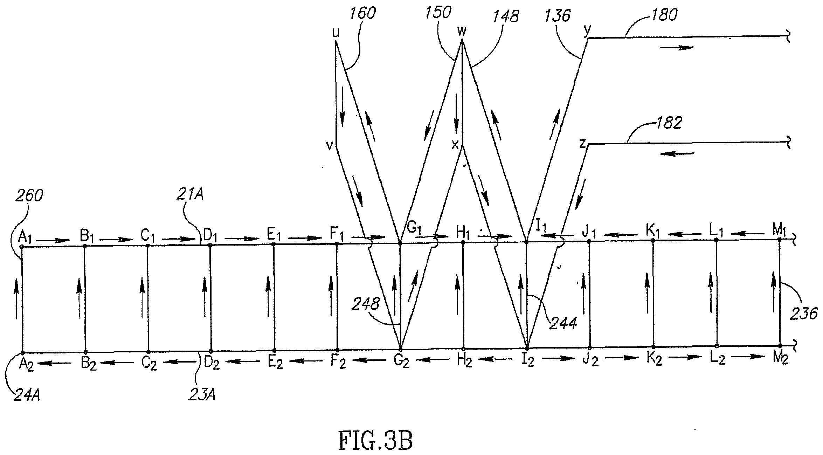

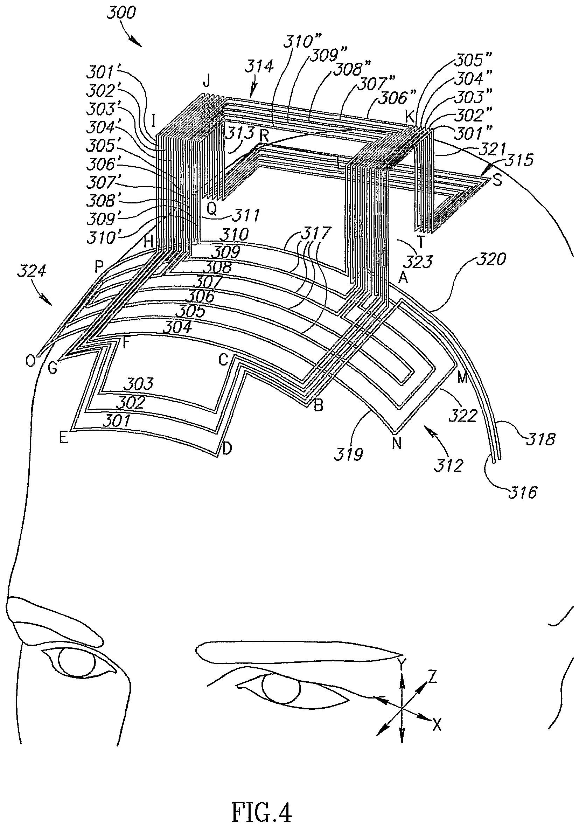

For purposes of better understanding the present invention, as illustrated in Figures 4-10 of the drawings, reference is first made to the construction and operation of previously described coils as illustrated in FIGS. 2A, 2B, 3A and 3B. The coils are shown in two different configurations, which have been previously disclosed in International Publication Number WO 02/32504, entitled, "Coil for magnetic stimulation and methods for using the same".

Referring now to FIG. 2A, a device 11 includes a frame and an electrically conductive coil having a base 12 and an outwardly projecting extension portion 14. In some embodiments, the frame itself is the electrically conductive coil, such as a frame composed of electrically conductive material. In other embodiments, however, the frame is a flexible or malleable material, which may be configured to a desired shape for a specific application, and the electrically conductive coil comprises one or more windings of electrically conductive material associated with the frame, such as being run alongside of, mounted to, wound around, or placed inside the frame. The base 12 has a concave first side 19, which is directed toward the body part of the subject, and a second side 20 opposite first side 19. The extension portion 14 extends outwardly from this second side and away from the base.

Device 11 can be placed in various orientations around the skull. However, device 11 effectively induces electric fields within the body of a subject when the device 11 is placed with the concave side 19 of the base 12 facing the body of the subject.

The device 11 pictured in FIG. 2A has a partially toroidal or ovate base 12 with a first end 22 and a second end 24. A line extending between these two ends 22, 24 defines a length axis along the length of the base 12. The base 12 has a substantially arcuate, semi-circular or semi-ovate shape along its length axis. The base 12 also has a width axis extending perpendicular to its length axis and this width axis has a substantially arcuate, semi-circular or semi-ovate shape. Thus, the base 12 pictured in FIG. 2A comprises an arch extending along its length axis and an arch extending along its width axis. The arch configurations along both the length and width axes are complementary to the external shape of the body part with which the device is to be used. The device conforms to the side-to-side and front-to-back arch shape of a subject's skull.

As shown in FIG. 2A, the base 12 includes a pair of substantially parallel, arcuate, elongate, longitudinally-extending, laterally spaced frame members 21 and 23. Extending between and interconnecting longitudinal frame members 21 and 23 are ten elongate, arcuate, transverse frame members 1, 2, 3, 4, 5, 6, 7, 8, 9, 10. Members 1-10 are spaced apart along the lengths of longitudinal frame members 21 and 23 and are coupled at their opposite ends to, and extend generally at right angles to, longitudinal frame members 21 and 23.

The extension 14 provides a path for the flow of electricity to and from the base 12. A surface charge can interfere with and reduce the strength of the electric field produced by the coil portions in the base. Reduction in surface charge is accomplished by using a triangular, or upwardly converging, extension 14. The extension 14 comprises first and second elongated elements 26, 28. The elements have a first set of inner ends 30, 32 connected to the base 12 at positions spaced apart along the length of the axis of the base 12. The first elongated element 26 has a first inner end 30 connected to the base 12 adjacent to the first end 22 of the base 12, and the second elongated element 28 has a first inner end 32 connected to the base 12 adjacent to the second end 24 of the base 12. The remainder portions 34, 36 of these elements 26, 28 extend away from the base 12 and converge toward each other.

FIG. 2B is a schematic diagram illustrating conducting wires and current flow in the example illustrated in FIG. 2A. In FIG. 2B, points labeled A- J and AA-JJ are associated with the base, and points Q-V are associated with the extension portion. Points U and V correspond to the electrical inputs for the current produced by the power supply (not shown). Using the diagram of FIG. 2B as a guide, one can understand how a coil might be constructed for the example of FIG. 2A. For example, the device 11 illustrated by FIG. 2A could comprise a coil having ten windings numbered 1-10 extending in the arch width direction of the base along the ten elongate, arcuate transverse frame members 1- 10. Table 1 summarizes such a placement of windings.

**Table 1**

| Winding No. | Pathway |
| --- | --- |
| 1 | V-R-H-I-J-JJ-II-H H-Q-U |
| 2 | V-R-H-I-II-HH-Q-U |
| 3 | V-R-H-HH-Q-U |
| 4 | V-R-F-G-GG-FF-Q-U |
| 5 | V-R-F-FF-Q-U |
| 6 | V-T-E-EE-S-U |
| 7 | V-T-E-D-DD-EE-S-U |
| 8 | V-T-C-CC-S-U |
| 9 | V-T-C-B-BB-CC-S-U |
| 10 | V-T-C-B-A-AA-BB-CC-S-U |

A significant portion of the current flowing through the base flows through the transverse strips of the coil and therefore, is oriented substantially along the reference z-axis shown in FIG. 2A. The coil portions associated with the base are complementary and tangential to the surface of the subject's skull. In particular embodiments, the total length of the coil associated with the transverse frame elements 1-10 (i.e. substantially parallel to the width axis of the base) exceeds the remaining length of the coil associated with the base (i.e. the remaining length substantially parallel to the length axis of the base). In these embodiments, a majority of the current flowing through the base is oriented substantially along the referenced z-axis shown in FIG.2A.

An alternative example of the device disclosed in International Publication Number WO 02/32504 is depicted in FIGS. 3A and 3B. A device 11A has a base 12A and an extension portion 14A, where base 12A has a first end 22A and a second end 24A, and a substantially arcuate, semi-circular or semi-ovate shape along its length and width axes. However, in this example, extension 14A includes a plurality of radially elongated extension elements 110, 112, 114,...158, 160, rather than a minimal number of radially elongated elements 26, 28 shown in FIG. 2A. The example shown in FIG. 3A includes twenty-six radially extending elongated extension elements 110, 112, 114,...158, 160, although alternative examples may employ a different number of such elongated extension elements. As illustrated, the radially elongated elements 110, 112, 114,...158, 160 are collected into four fan-like groupings 170, 172, 174, 176, and elongated elements 134 and 136 are connected by lateral elements 180 and 182.

Similar to base 12 illustrated in FIG. 2A, base 12A illustrated in FIG. 3A includes a pair of substantially parallel, arcuate, elongate, longitudinally- extending, laterally spaced frame members 21A and 23A. Extending between and interconnecting longitudinal frame members 21A and 23A are twenty-six elongate, arcuate transverse frame members 210, 212, 214, ... 258, 260.

The amount of surface charge, and the influence of that surface charge on the deeper tissues of the subject's body that are stimulated, depends on the overall lengths and locations of the electrical components which contain non-tangential components. In this embodiment, the overall lengths of such non-tangential elements are reduced and their distances from the deep brain regions aimed for activation are increased. In other words, the ratio of the total length of the coil extending radially from the base to the total length of the coil associated with the base is less than the corresponding ratio in the previous embodiment, shown in FIG. 2A.

FIG. 3B is a schematic illustration of current flow through the windings of the embodiment illustrated in FIG. 3A, with reference numerals correlating these windings to certain structures illustrated in FIG. 3A. FIG. 3B is not a circuit diagram in the true sense -this illustration simply shows how a coil for the device may be made from individual windings of the coil, with each individual winding comprising a circuit. For the sake of clarity, only part of the entire device is shown.

As illustrated in FIG. 3B, the direction of electrical current flow is the same in all of the twenty-six strips of the base 12A, flowing in a direction from the lateral frame member 23A to lateral frame member 21A. Generally, current to this portion of the coil arrives at Z, travels down to $I_2$, and flows through strips $J_2$-$J_1$, $K_2$-$K_1$, $L_2$-$L_1$, and $M_2$-$M_1$. Each strip ($A_2$-$A_1$, $B_2$-$B_1$,...$M_2$-$M_1$) has a return path through an elongated element 110, 112, 114, ...158, 160 of one of the fan-like groupings 170, 172, 174, 176. For example, the return path for strip $J_2$-$J_1$ may be elongated element 140 (not shown in FIG. 3B). The current flows to $I_2$ then flows through strip $H_2$-$H_1$, and to $I_1$. From here, the current flows up the extension to W, then to X (the line W-X representing the junction of two elongated elements 148 and 150), then to $G_2$, then through strips $F_2$-$F_1$, $E_2$-$E_1$, $D_2$-$D_1$, $C_2$-$C_1$, $B_2$-$B_1$, $A_2$-$A_1$, and returns to $G_2$. Each of strips $F_2$-$F_1$, $E_2$-$E_1$, $D_2$-$D_1$, $C_2$-$C_1$, $B_2$-$B_1$, $A_2$-$A_1$, has a return path through an elongated element of fan-like collection 176 composed of elongated elements 150-160. The return paths of current flow are in the opposite directions of the strips. As in the first example, shown in FIG. 2A, extension portion 14A of this second embodiment places electrical currents flowing through the return paths away from the subject, to reduce their electrical effect on the body tissues of the subject.

In the two previously described examples of a device for magnetic stimulation described above with reference to FIGS. 2A-B and 3A-B, return paths are placed away from the subject, to reduce their electrical effect on the body tissues of the subject. However, increasing the distance from the skull requires longer non-tangential elements and causes an accumulation of surface charges, which increases the decay in electrical field with depth. These conflicting principles are balanced as much as possible, so as to minimize both unwanted electrical effects due to current flow in the return paths and unwanted accumulation of surface charges.

In the present application, a design to further decrease the lengths of non-tangential elements (and thus minimize unwanted surface charges at the area of stimulation) is disclosed. The embodiments described herein are particularly useful in cases where the region to be stimulated is not on a central line of the brain, such as prefrontal regions.

Reference is now made to FIG. 4, which is an illustration of a coil 300 for TMS in accordance with one preferred example of the present disclosure. Coil 300 includes a base portion 312, a protruding return portion 314, and a contacting return portion 315. Base portion 312 is comprised of windings 317 of electrically conductive material. Base portion 312 has a concave first side 319, which is in direct contact with the skull and is directed toward the body part of the subject, and a second side 320 opposite first side 319. Protruding return portion 314 extends outwardly from second side 320 and away from base portion 312, and contacting return portion 315 is positioned a distance from base portion 312, but is in contact with the skull. Thus, base portion 312 can be considered to be at a first height with respect to the target area. Protruding return portion 314 is at a second height wherein the first and second heights are defined with respect to the y-z plane . Contacting return portion 315 is at the first height, that is, is approximately on the same plane as base portion 312, but is at a planar distance (in the x-z plane) from the target area. Windings 317 are designed to be in contact with the skull, and may either be pre-formed or malleable to accommodate the curved anatomy of the area on which it is to be placed. This design maximizes tangential stimulation, which is optimal for axonal depolarization. 5

The device 300 pictured in FIG.4 has an arcuate base 312 with a first end 322 and a second end 324. A line extending between these two ends 322, 324 defines a length axis along the length of the base 312. The base 312 has a substantially arcuate, semi-circular or semi-ovate shape along its length axis. The base 312 also has a width axis extending perpendicular to its length axis and this width axis has substantially arcuate, semi-circular or semi-ovate shape. Thus, the base 312 pictured in FIG. 4 comprises an arch extending along its length axis and an arch extending along its width axis. The arch configurations along both the length and width axes are complementary to the external shape of the body part with which the device is to be used. The device conforms to the side-to-side and front-to-back arch shape of a subject's skull.

Base 312 includes windings 317, which are comprised of a series of substantially parallel members 301-310. In the embodiment depicted in FIG. 4, members 301-310 are oriented in a lateral-medial direction, making device 300 suitable for activating structures in the prefrontal cortex and fibers connecting the 20 cingulate or prefrontal cortex with the nucleus accumbens and ventral tegmental area. These are neuronal pathways related to the control of motivation, reward and pleasure. Each of members 301-310 carries an electrical current in the lateral-medial direction (substantially parallel with the length axis of base 312), with the direction of the current being the same in each of members 301-310. Each of members 301-310 has a return path, extending through either protruding return portion 314 or through contacting return portion 315. The members 301-310 are electrically connected to a power supply, such as by electrical leads 316, 318. In a preferred embodiment, each of members 301-310 is 14-22 cm in length, in one embodiment, there is a separation of 0.5-1.5 cm between each of members 301-310. In a preferred embodiment, there is a separation of 0.8 cm between each of members 301-310. The return paths 306"-310" of members 306-310 are situated above the head at a distance therefrom as delineated by segments H-I. In one example, the distance from the head to the return paths 306"-310" of members 306-310 is between 4-10 cm. In a preferred example, the distance from the head to the return paths 306"-310" of members 306-310 is approximately 7 cm.

Coil 300 may be composed of any electrically conductive material, such as metal. Particular examples have coils comprising wire made of copper, aluminum, or other electrically conductive material. In a preferred examples, the coil is made of a double 14 AWG insulated copper wire having a total length of 800 cm and winded into windings 317, connected in series. In alternative embodiments the coil is made of 7 Shelamid 200 copper wires insulated by two polyester layers (66 [mu]m insulation width), with 0.9 mm diameter of each wire, or any kind of multi-line wires. In another example the coil is made from a multiline wire composed of 40-60 lines of 3 mm cross section. In a preferred example, coil elements are coated by a polyurethane resin type Resinex 4 (Hamchaber Vehamkasher Ltd., Israel), for additional electrical insulation. In alternative embodiments coil elements are coated by a RNF-3000 heat-shrinkable tube with 0.65 mm thickness (Raychem Corp, Menlo Park, CA, USA), for additional electrical insulation. In alternative examples, coil elements are coated by other insulating materials, such as PVC, or are sandwiched between layers of insulating materials. It should be readily apparent that the examples disclosed herein should not be regarded as limiting. The windings 317 are connected to an appropriate cable and connector, which is then connected to a stimulator. The stimulator may be any appropriate commercially available power supply, such as the power supplies available for use with other magnetic coils. In some examples, the stimulator is one of various models of magnetic stimulators produced by Medtronic, Inc. of Minneapolis, MN, USA (e.g., MagPro, MagLite Compact), or power supplies sold with various models of magnetic stimulators produced by Magstim Company US, LLC, of New York, NY, USA (e.g., Magstim Model 200, Magstim Model 220, Magstim Model 250, BiStim, Magstim Rapid, Magstim QuadroPulse Magstim Rapid<2>).

A power supply or stimulator (not shown) supplies current through lead 316 into one of members 301-310. The stimulating current pulses flow substantially in the lateral-medial direction. Current then ascends through an ascending portion 311 extending upwards from base portion 312. At this point, current can take one of two paths - either through protruding return portion 314 or through contacting return portion 315. If current runs through protruding return portion 314, it runs from ascending portion 311, through protruding return portion (which runs substantially parallel to members of base portion 312), and back down to the level of the skull at a descending portion 323. From there, current returns through lead 318 back into the power supply. If current runs through contacting return portion 315, it runs from ascending portion 311, to a descending connector 313, through contacting return portion 315 (which runs substantially parallel to members of base portion 312 and is positioned directly on the skull, but at a distance from members 301-310 of base portion 312), to an ascending connector 321 , and back down to the level of the skull at descending portion 323. From there, current returns through lead 318 back into the power supply. In a preferred example, half of the members run through protruding return portion 315 and half of them run through contacting return portion 314. However, the invention is not limited to this proportion, and any proportion of protruding return paths and contacting return paths is possible, so long as each return path receives current from at least one of the members. Current may be supplied simultaneously to all members, or alternatively, may be supplied sequentially, in a random order, or selectively. In another example, current is supplied to member 301, and runs through a loop including each of the additional members 301-310. It should also be readily apparent that although the invention has been shown with reference to ten members, the invention is not in any way limited to this number, and any suitable number of members may be used. In additional examples, a single member may have a return path through both protruding return portion 314 and contacting return portion 315. In additional examples each member may have several wires connected in series, while the different members may be connected in parallel, and activated either sequentially, simultaneously, or in any sequence.

In the preferred example depicted in FIG. 4, current from each of members 301-310 runs through ascending portion 311 via pathways 301'- 310'. At the top of ascending portion 311, current from members 301-305 runs through contacting return portion 315 via pathways 301"-305" while current from members 306-310 runs through protruding return portion 314 via pathways 306"- 310". Specifically, members 301-303 traverse the path A-B-C-D-E-F-G-H-I-J-Q-R-S-T-K-L-A. Member 304 traverses the path A-B-G-H-I-J-Q-R-S-T-K-L-A. Member 305 traverses the path A-M-N-B-G-O-P-H-I-J-Q-R-S-T-K-L-A. Members 306-307 traverse the path A-M-N-B-G-O-P-H-I-J-K-L-A. Members 308-309 traverse the path A-B-G-H-I-J-K-L-A. Member 310 traverses the path A-H-I-J-K-L-A. It should be readily apparent that other combinations and pathways are possible, and are within the scope of the present invention.

Protruding return portion 314 is spaced a distance from the skull, as described above. By placing the return path at a distance from the skull, electrical stimulation of unwanted portions of the brain is minimized. However, surface charge accumulation at the surface of the brain is increased. As such, some of the return paths are placed on the skull itself, so as to reduce surface charge accumulation. However, these return paths are placed a distance from the site to be stimulated within the brain so as to avoid conflicting signals in the area of stimulation. In a preferred example, the distance from the members to the contacting return paths is at least 5 cm. In some examples, the distance from the members to the contacting return paths is in the range of 7-20 cm. In a preferred example, the distance is approximately 10 cm. Thus, a balance is maintained between the need for reducing surface charge and the conflicting need to avoid electrical stimulation of unwanted portions of the brain.

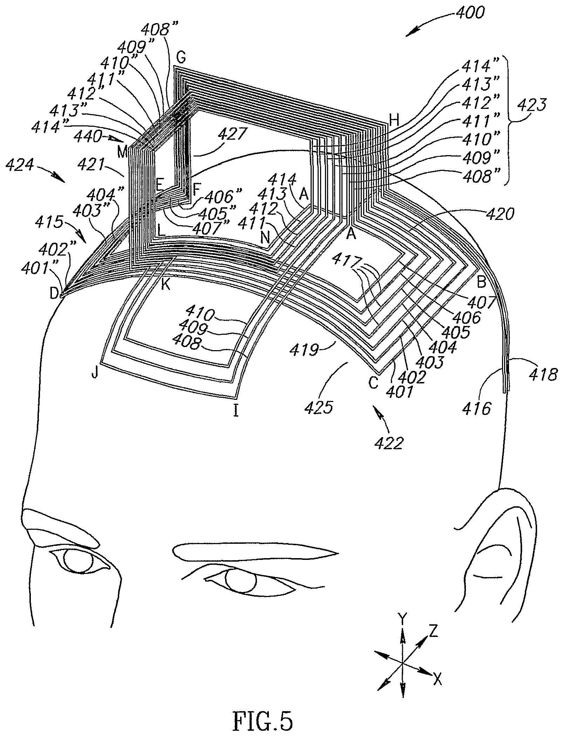

Reference is now made to FIG. 5, which is an illustration of a coil 400 for TMS in accordance with another preferred example of the present disclosure. In this example, members 401-414 are oriented in an anterior- posterior direction, for activation of structures in the prefrontal cortex and fibers connecting the cingulate or prefrontal cortex with the nucleus accumbens and ventral tegmental area, with preference for the left hemisphere These are neuronal pathways related to the control of motivation, reward and pleasure. Coil 400 includes a base portion 425, a protruding return portion 440, and a contacting return portion 415. Base portion 425 is comprised of windings 417 of electrically conductive material. Base portion 425 has a concave first, or inner side 419, which is in direct contact with the skull and is directed toward the body part of the subject, and a second, or outer side 420 opposite first side 419. Protruding return portion 440 extends outwardly from second side 420 and away from base portion 425, and contacting return portion 415 is positioned a distance from base portion 425, but is in contact with the skull. Thus, base portion 425 can be considered to be at a first level with respect to the target area. Protruding return portion 440 is at a second level which is at a distance from the first level in the $\gamma$-direction. Contacting return portion 415 is at the first level, that is, is approximately on the same plane as base portion 425, but is at a planar distance (in the x-z plane) from the target area. Windings 417 are designed to be in contact with the skull, and may either be pre-formed or malleable to accommodate the curved anatomy of the area on which it is to be placed. This design maximizes tangential stimulation, which is optimal for axonal depolarization.

The device 400 pictured in FIG. 5 has an arcuate base 425 with a first end 422 and a second end 424. A line extending between these two ends 422, 424 defines a length axis along the length of the base 425. The base 425 has a substantially arcuate, semi-circular or semi-ovate shape along its length axis. The base 425 also has a width axis extending perpendicular to its length axis and this width axis has substantially arcuate, semi-circular or semi-ovate shape. Thus, the base 425 pictured in FIG. 5 comprises an arch extending along its length axis and an arch extending along its width axis. The arch configurations along both the length and width axes are complementary to the external shape of the body part with which the device is to be used. The device conforms to the side-to-side and front-to-back arch shape of a subject's skull.

Base 425 includes windings 417, which are comprised of a series of substantially parallel members 401-414. In the example depicted in FIG. 5, members 401-414 are oriented in an anterior-posterior direction, making device 400 suitable for activating structures in the prefrontal cortex. Each of members 401-414 carries an electrical current in the anterior-posterior direction (substantially perpendicular with the length axis of base 425), with the direction of the current being the same in each of members 401-414. Each of members 401- 414 has a return path, extending through either protruding return portion 440 or through contacting return portion 415. The members 401-414 are electrically connected to a power supply, such as by electrical leads 416, 418. In a preferred example, each of members 401-414 has a length of 7-12 cm. The 14 members 401-414 are distributed above the prefrontal cortex of the left hemisphere. In one example, there is a separation of 0.5-1.5 cm between each of members 401-414. In a preferred example, there is a separation of 1 cm between each of members 401-414. Three members 408-410 are elongated towards the forehead, and their continuations pass in the left-right direction along the orbitofrontal cortex to provide additional effects in that region, as delineated by segments I-J. Return paths 401"-407" of members 401-407 are attached to the head in the right hemisphere, as delineated by segments D-E. In one example, each of the return paths 401"-407" is separated from one another by approximately 0.5-1.2 cm. In a preferred example, each of the return paths 401"-407" is separated from one another by approximately 0.8 cm. The return paths 408"-414" of members 408-414 are situated above the head at a distance therefrom as delineated by segments M-G. In one example, each of the return paths 408"-414" is separated from one another by approximately 0.1-0.7 cm. In a preferred example, each of the return paths 401"-407" is separated from one another by approximately 0.3 cm. In one example, the distance from the head to the return paths 408"-414" of members 408-414 is between 4-10 cm. In a preferred example, the distance from the head to the return paths 408"-414" of members 408-414 is approximately 7 cm.

Coil 400 may be composed of any electrically conductive material, such as metal. Particular examples have coils comprising wire made of copper, aluminum, silver, or other electrically conductive material. In a preferred example, the coil is made of a double 14 AWG insulated copper wire having a total length of 750 cm and winded into windings 417, connected in series. In alternative examples the coil is made of 7 Shelamid 200 copper wires insulated by two polyester layers (66 [mu]m insulation width), with 0.9 mm diameter of each wire, or any kind of multi-line wires. In a preferred example, coil elements are coated by a polyurethane resin type Resinex 4 (Hamchaber Vehamkasher Ltd., Israel), for additional electrical insulation. In alternative examples, coil elements are coated by other insulating materials, such as PVC, or are sandwiched between layers of insulating materials. In alternative examples coil elements are coated by a RNF-3000 heat-shrinkable tube with 0.65 mm thickness (Raychem Corp, Menlo Park, CA, USA), for additional electrical insulation. It should be readily apparent that the examples disclosed herein should not be regarded as limiting. The windings 417 are connected to an appropriate cable and connector, which is then connected to a stimulator. The stimulator may be any appropriate commercially available power supply, such as the power supplies available for use with other magnetic coils. In some examples, the stimulator is one of various models of magnetic stimulators produced by Medtronic, Inc. of Minneapolis, MN, USA (e.g., MagPro, MagLite Compact), or power supplies sold with various models of magnetic stimulators produced by Magstim Company US, LLC, of New York, NY, USA (e.g., Magstim Model 200, Magstim Model 220, Magstim Model 250, BiStim, Magstim Rapid, Magstim QuadroPulse, Magstim Rapid<2>).

The stimulator or power supply (not shown) supplies current through lead 416 into one of members 401-414. The stimulating current pulses flow substantially in the anterior-posterior direction. At this point, current can take one of two paths - either through contacting return portion 415 or through an ascending portion 421 extending upwards from base portion 425 and then through protruding return portion 440. If current runs through contacting return portion 415, it runs from base portion 425 to contacting return portion 415 (which runs substantially parallel to members of base portion 425, and is positioned directly on the skull but at a distance from members 401-414 of base portion 325) through an ascending connector 427 and back down to the level of the skull via descending portion 423. From there, current returns through lead 418 back into the power supply. If current runs through protruding return portion 440, it runs from ascending portion 421, through protruding return portion 440 (which runs substantially parallel to members of base portion 425), and back down to the level of the skull at a descending portion 423. From there, current returns through lead 418 back into the power supply. In a preferred example, half of the members run through protruding return portion 440 and half of them run through contacting return portion 415. However, the invention is not limited to this proportion, and any proportion of protruding return paths and contacting return paths is possible, so long as each return path has current from at least one of the members. Current may be supplied simultaneously to all members, or alternatively, may be supplied sequentially, in a random sequence, or selectively. In another example, current is supplied to member 401 , and runs through a loop including each of the additional members 401-414. It should also be readily apparent that although the invention has been shown with reference to fourteen members, the invention is not in any way limited to this number, and any suitable number of members may be used. In additional examples, a single member may have a return path through both protruding return portion 440 and contacting return portion 415.

In the preferred example depicted in FIG. 5, current from each of members 401-407 runs through contacting return portion 415 via pathways 401"-407" while current from members 408-414 runs through ascending portion 421 and through protruding return portion 440 via pathways 408"-414". Specifically, members 401-407 traverse the path A-B-C-D-E-F-G-H-A. Members 408-410 traverse the path A-I-J-K-L-M-G-H-A. Members 411-414 traverse the path AN-L-M-G-H-A. It should be readily apparent that other combinations and pathways are possible, and are within the scope of the present invention.

Protruding return portion 414 is spaced a distance from the skull. In One example, this distance is in a range of 4-10 cm. In a preferred example, this distance is 7 cm. By placing the return path at a distance from the skull, electrical stimulation of unwanted portions of the brain is minimized. However, surface charge accumulation at the surface of the brain is increased. As such, some of the return paths are placed on the skull itself, so as to reduce surface charge accumulation. However, these return paths are placed a distance from the site to be stimulated within the brain so as to avoid conflicting signals in the area of stimulation. In one example, the distance from the central members (such as member 414, for example) to the contacting return paths is in a range of 7-15 cm. In a preferred example, the distance from the central members (such as member 414, for example) to the contacting return paths is approximately 8 or 9 cm. Thus, a balance is maintained between the need for reducing surface charge and the conflicting need to avoid electrical stimulation of unwanted portions of the brain.

In one example, a screen may be applied to either of coils 300 or 400 to further reduce the magnetic field produced when electricity runs through the return portions. The screen is comprised of a material with high magnetic permeability, capable of inhibiting or diverting a magnetic field, such as mu metal, iron or steel. Alternatively the screen is comprised of a metal with high conductivity which can cause electric currents or charge accumulation that may oppose the effect produced by the return portions. Any suitable screen or shield capable of inhibiting magnetic fields may be used. The screen may be any suitable size or shape, including but not limited to sheaths of mu metal surrounding one, some or all of the members of coil 300 or 400, a flat disc of metal strategically placed, or an enclosure substantially enclosing the return paths.

Reference is now made to FIG. 6, which is a block diagram illustration of a cooling system 88, in accordance with one example of the present disclosure. Although the example described herein refers to water or other liquids used for cooling, it is envisioned that air cooling may be used. The term "fluid" herein denotes liquid such as water, or gas such as a mixture of gases and more specifically, air. Cooling system 88 is designed for maintaining ambient temperature in the coils during repetitive operation. Cooling system 88 includes an external cooling unit 500, a fluid circulator 502, and an internal system 504. Internal system 504 is connected to coil 300 or 400. Arrows 506 represent the direction of cooling.

Reference is now made to FIG. 7, which is a block diagram illustration of external cooling unit 500. External cooling unit 500 includes a compressor 506, a condenser 508, an expansion valve 510, and a carburetor 512. Compressor 506 is a commercially available compressor (available, for example, from Electrolux, Thailand, Type L57TN). In a preferred example, condenser 508 is made of 3/8 inch diameter pipe, and has 0.5 horse power, a ventilator with 5-30W engine (EMI, Italy), and current of up to 0.20 A. Expansion valve 510 is made of capillary pipe having approximately a 0.07 inch diameter and a length of 4 meters. Carburetor 512 is made of a 3/8 diameter spiral pipe having a total length of at least 4.7 meters.

Reference is now made to FIG. 8, which is a block diagram illustration of fluid circulator 502. In the example described herein, fluid circulator is a water circulator, and includes a water tank 514 and a water pump 516. Water tank 514 is in contact with carburetor 512 of external cooling unit 500. In a preferred example, water tank 514 is a 10 liter iron tank coated by a 1 cm layer of foamed polyurethane. Water pump 516 is in fluid communication with internal system 504, and is configured to deliver cooled water to radiators of internal system 504. Water pump 516 is a commercially available water pump available, for example, from Pentax, Italy (Type CM50/01). The nominal working pressure used is 2 bar. The pressure is regulated by a manual feedback cock. The excess of water returns to the tank and creates circulation.

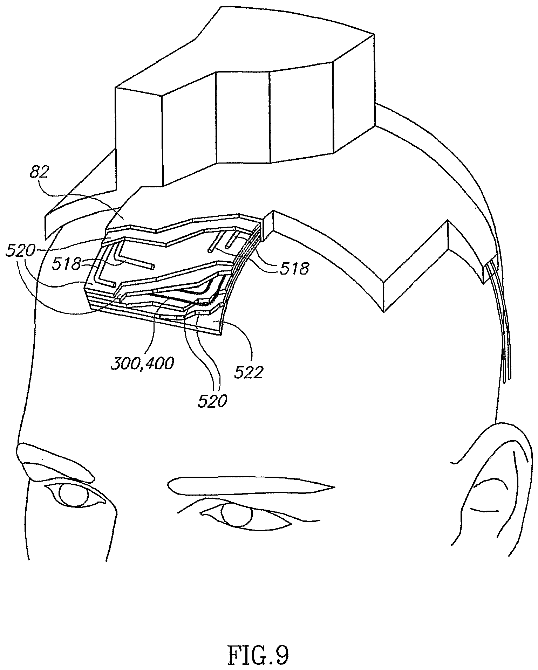

Cooling is accomplished as follows. Freon gas is compressed in compressor 506, condensed in condenser 508, and expanded through expansion valve 510. The capillary in expansion valve 510 is connected to carburetor 512, where the gas is evaporated again and returns to compressor 506. Carburetor 512 is immersed in water tank 514, thereby cooling the water. The water is pumped out via water pump 516, and circulated through radiators of internal system 504. In alternative examples, cooled air is circulated instead of water. In one example, internal system 504 is a radiator system. Radiators are in thermal conjunction with coil 300 or 400, as will be described in greater detail hereinbelow with reference to FIG. 9. The fluid circulation cools the coil during pulse trains and stabilizes its temperature at mild temperature range. In one example, temperature sensors are located at or near coil 300 or 400, and information about temperature during a procedure can be sent directly to cooling system 88. Automatic adjustment of cooling can then be done based on the temperature information.

Reference is now made to FIG. 9, which is a schematic illustration of internal system 504 in contact with coils 300 or 400, in accordance with one example of the present disclosure. In the example shown herein, internal system 504 includes individual radiator units 518 (shown partially cut), in close thermal contact and approximate geometric alignment with coils 300 or 400. Each of radiator units 518 are comprised of two parallel 1/4 inch pipes, and between them several capillary pipes of 0.07 inch diameter. In a preferred example, the pipes are made of copper and are coated by insulating lacquer (John C. Dolph Company, New Jersey, USA). Radiator units 518 are sandwiched by layers of a thermal and electrical insulator 520. Coil 300 or 400 is also sandwiched by layers of insulator 520. In a preferred example, insulator 520 is a semi-flexible polyurethane resin, preferably Resinex 4 available from Hamchaber and Hamkasher Ltd., Bat Yam, Israel. In a preferred example, at least two layers of insulator 520 are situated on either side of coil 300 or 400, and at least one layer of insulator 520 is further situated between radiator units 518 and helmet 82. An additional layer of biocompatible foam medical tape 522 (for example, Type 9776 available from 3M Center, St. Paul, MN, USA) attaches the entire system to the head of the subject. The number of radiator units 518 depends on the number of members or coil units in the coil. For example, for coil 300, six radiator units are used, and for coil 400, seven radiator units are used.

Methods of Operation (not forming part of the present invention):

The basic method for operating system 80 of the present invention involves the following steps: First, subjects are fitted with earplugs to lessen any possible adverse effect on hearing. The subject is then seated on chair 85 with his/her heading resting on rear head support 87. Helmet 82 with coil 300 or 400 and radiator units 518 or with any other suitable cooling system is positioned over the subject's head over the prefrontal cortex, 5 cm anterior to the hot spot for abductor pollicis brevis (APB) muscle stimulation. The subject's motor threshold is measured by delivering single stimulations to the motor cortex, by gradually increasing the intensity (using the single pulse mode, applying one pulse each time) and recording electrical activity in abductor pollicis brevis (or any other muscle) using surface electrodes. Threshold is defined as the lowest intensity of stimulation able to produce motor evoked potentials of at least 50[mu]V in 5 of 10 trials. After defining the motor threshold, coil 300 or 400 is positioned on the prefrontal cortex, and the session is performed at 110% of the motor threshold. Stimulator 86 is set to required power, frequency and duration values, as determined. Frequency can range from 1-50 Hz.

Each treatment session includes a predetermined number of trains. In some examples, a train of 1 to 100 pulses is administered. Individual pulses measure from about 50 to 2000 microseconds, preferably in the 1000 microsecond range. In an example, the duration of each train is 1 second, with an inter-train interval of 20 seconds. Alternative durations and intervals are possible as well. Treatment plans can include, for example, an increase in the frequency used on different days. Pulses can vary in frequency as well as number.

In a preferred example, each treatment session includes 42 trains. The duration of each train is 1 second and the inter-train interval is 20 seconds. Each subject undergoes three treatment sessions, on day 1 , 3 and 5. On day 1, stimulation is 1 Hz, on day 2, stimulation is 10 Hz, and on day 3, stimulation is 20 Hz.

The basic principles and operation of the system is based on summation of electrical impulses. The general concept of summation is that by providing several sub-threshold impulses, it is possible to stimulate deep regions of the brain without unwanted stimulation or excessive electrical field applied at surface areas of the brain. In International Publication Number WO 02/32504, this concept was applied spatially by using several coil elements carrying current in a desired direction, each placed in a different location around the head such that high electric field intensity is concentrated in a specific deep brain region, while maintaining a high ratio of deep brain electrical field to surface electrical field. This type of spatial summation can be termed one-point spatial summation, since each of the individual elements stimulates the same focused point.

While one-point spatial summation has been shown to be advantageous, other more specific methods could be useful in further increasing the depth penetration and specificity of the treatment.

In one embodiment of the present invention, a different type of spatial summation is contemplated. Rather than focusing on a single point, several points along a neuronal structure can be stimulated, causing a net result depolarization at an even lower electrical field strength. This type of spatial summation can be termed morphological line spatial summation. The points along the neuron at which the electric field is produced may or may not be in a straight line configuration. If, for example, a path of a specific axonal bundle is known, such as for example the medial forebrain bundle, the coil can be designed in a configuration to produce significant electrical fields at several points along the bundle. The configuration of the coil would approximate the path of the bundle, which can be determined, for example, by a fiber tracking diffusion tensor MRI or by other known imaging methods. This configuration may enable induction of an action potential in the bundle, while minimizing activation of other brain regions. Specifically, the coil can be activated at an intensity which is sub-threshold and thus would not induce an action potential at one specific brain region, but since it is being induced along a specific path, the summation of points in space would be enough to induce the action potential in the desired axonal bundle.

Time Summation Principle

In general, TMS stimulators may produce a monophasic or a biphasic pulse of electric current. During the discharge cycle, the TMS circuit behaves like a RCL circuit, and the current I is given by:

$$I(t) = \frac{(\underline{V})\exp(-\alpha t)\sin(wt)}{wL} \qquad (1)$$

where V is the maximal voltage, $\alpha = R/2L$, $w = \sqrt{((LC)^{-1} - \alpha^2)}$, and R, C and L are the total values of the resistance, capacitance and inductance, respectively, in the circuit. The inductance is mainly the coil inductance, but there is an additional contribution from the cables, and the resistance includes contributions from a switch (as described below) and the coil.

The total energy on the capacitor before discharging is given by:

$$W_c = \frac{1}{2}CV^2 \qquad (2)$$

In principle two related parameters may be relevant for neuronal activation: The electric field strength, and the spatial derivative of the electric field. While the activation of peripheral nerves depends mainly on the derivative of the electric field along the nerve fiber, for neuronal tissue with relatively short axons with bends and branches, such as the brain, the absolute magnitude of the induced electric field is the biologically relevant parameter for neuronal stimulation. The induced electric field is proportional to the rate of change of current (dI/dt). The brief strong current generates a time-varying magnetic field B. An electric field E is generated in a direction which is perpendicular to the magnetic field, and has an amplitude which is proportional to the time-rate change of the vector potential A(r).

The vector potential A(r) in position r is related to the current in the coil I by the expression:

$$A(r) = \frac{\mu_0 I}{4\pi} \int \frac{dl`}{|r-r`|} \qquad (3)$$

Where $\mu_0 = 4\pi * 10^{-7}$ Tm/A is the permeability of free space, the integral of dl' is over the wire path, and r' is a vector indicating the position of the wire element. The magnetic and electric fields are related to the vector potential through the expressions:

$$B_A = \nabla \times A \tag{4}$$

$$E_A = -\frac{\partial A}{\partial t} \tag{5}$$

The only quantity which changes with time is the current I. Hence the electric field $E_A$ can be written as:

$$E_A = -\frac{\mu_0}{4\pi}\frac{\partial I}{\partial t}\int \frac{dl`}{|r-r`|} \tag{6}$$

Since brain tissue has conducting properties, while the air and skull are almost complete insulators, the vector potential will induce accumulation of electric charge at the brain surface. This charge is another source for electric field, which can be expressed as:

$$E_\Phi = -\nabla\Phi \tag{7}$$

Where $\Phi$ is the scalar potential produced by the surface electrostatic charge. The total field in the brain tissue E is the vectorial sum of these two fields:

$$E = E_A + E_\Phi \tag{8}$$

The electrostatic field $E_\Phi$ generally opposes the induced field $E_A$, which consequently reduces the total field E. The amount of surface charge produced and hence the magnitude of $E_\Phi$ depends strongly on coil configuration and orientation.
Referring back to Eq. 1, in most practical TMS circuits with accepted values of R, C and L, the condition:

$$\alpha << w \tag{9}$$

is fulfilled. Since the electric field is proportional to the time derivative of the current, its time dependence can be approximated by:

$$E = E_o \exp(-\alpha t)\cos(wt) \tag{10}$$

This electric field produces an action potential in excitable neuronal cells, which may result in activation of neuronal circuits if a particular threshold is reached. The neuronal response depends not only on the electric field strength, but also on the pulse duration. The duration $\tau$ of one pulse cycle is about:

$$\tau = 2\pi\sqrt{(LC)} \tag{11}$$

The longer the pulse duration, the smaller the required electric field to reach neuronal threshold $E_{thr}$. The dependence of $E_{thr}$ on pulse duration is given by a strength-duration curve of the form:

$$E_{thr} = b(1 + 2c/\tau) \tag{12}$$

where b is the Rheobase, which corresponds to the electric field required to induce neuronal activation at infinite duration, and c is the Chronaxie, related to the time constant of neuronal membrane (as described, for example, in Bourland JD, Nyenhuis JA, Noe WA, Schaefer JD, Foster KS, and Geddes LA, in Proc. Int. Soc. Magnetic Resonance in Medicine 4th Scientific Meeting, New York, 1996, p 1724).

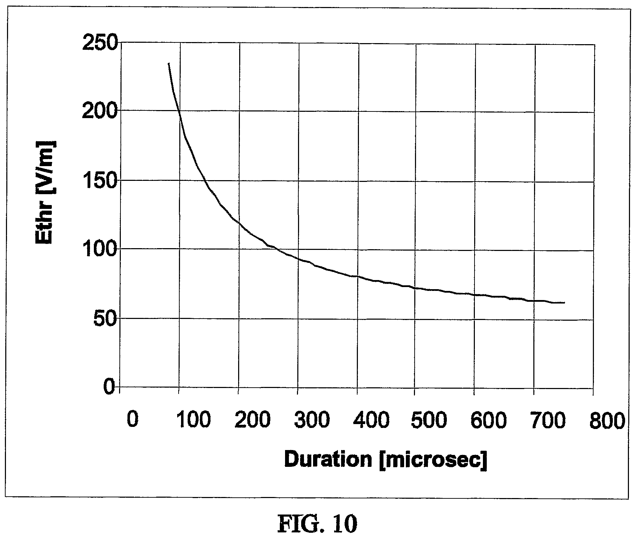

Reference is now made to Fig. 10, which is a strength-duration curve reflecting the average of four subjects, using eight different coils with inductance L of between 6 and 148 $\mu$H.

As can be seen from Eq. 11, the duration of a TMS pulse can be extended in two ways: by increasing the capacitance C, or by increasing the coil inductance L. In known stimulators, C is constant. Increasing L leads to increased power and energy consumption. From Eq. 1 it can be seen that the peak induced electric field $E_{max}$ is proportional to:

$$E_{max} \; \alpha \; dI/dt \; \alpha \; V/L \qquad\qquad (13)$$

The electric field required to reach neuronal threshold, $E_{thr}$, decreases with increasing L (Eqs. 11 and 12). Examining the ratio, r, between the induced peak electric field and the required threshold, by combining Eqs. 11-13, we obtain:

$$r = E_{max}/E_{thr} = \frac{E_0 \, V/L}{b(1+2c/(2\pi\sqrt{(LC)})} = \frac{E_0 \, V/L}{((a_1\sqrt{(LC)}+a_2)/\sqrt{(LC)})} = \frac{E_0 \, V\sqrt{C}}{(a_1\sqrt{(C)}L+a_2\sqrt{(L)})} \qquad (14)$$

where Eo is a pre-factor depending on various parameters including coil geometry, configuration, inductance, number of turns etc. $a_1$ and $a_2$ were introduced in order to emphasize the dependence on L and C. It can be seen that the voltage required to reach neuronal activation threshold increases with L, and slowly decreases with C.

In order to obtain the dependence of the required energy on L and C, we substitute r=1 in Eq. 14 and then substitute V in the expression for the total capacitor energy (Eq. 2):

$$W_c = \tfrac{1}{2}CV^2 = \frac{\tfrac{1}{2}L(a_1^2 LC + 2a_1a_2\sqrt{(LC)} + a_2^2)}{E_0^2} \qquad\qquad (15)$$

It can be seen that the required energy increases quadratically with L and linearly with C.

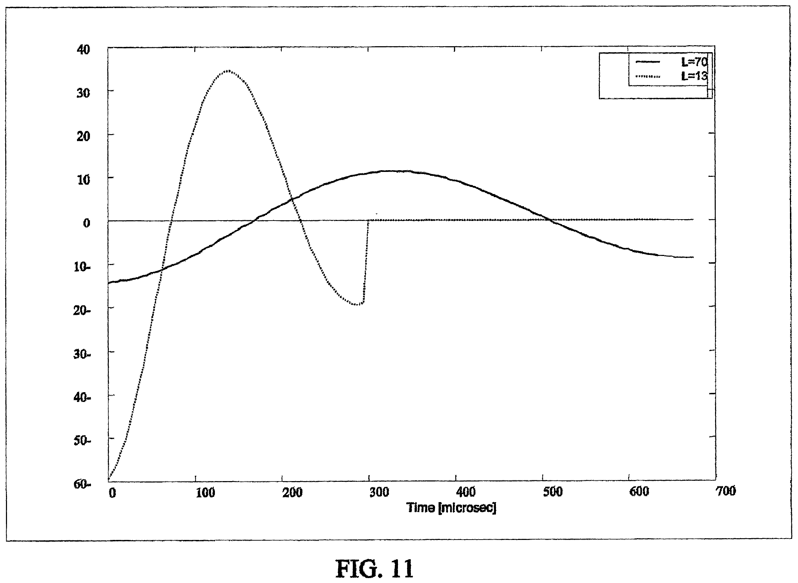

Reference is now made to Fig. 11, which depicts pulses produced by TMS coils having inductances of 13 and 70 $\mu$H. The amplitudes represent the threshold electric field according to points 1 and 2 in the strength-duration curve shown in Fig. 10. The plots are for resistance and capacitance of R=0.1 Ohm and C= 165 $\mu$f, respectively.

It can be seen that when the pulse duration is longer, the required threshold electric field is smaller.

In regular TMS stimulators, the capacitor (or bank of capacitors) is discharged through a single switch to a single coil, resulting in simultaneous current flow through all coil elements, and thus simultaneous electric fields produced by all coil elements. Thus, the electric field induced in cortical brain regions close to coil elements will in general be larger than the field induced in deeper brain regions.

In one embodiment of the present invention, each of the various coil members can be stimulated consecutively, resulting in temporal summation. Since neuronal activation threshold depends on both the strength / intensity of the electric field and the stimulation duration, the threshold may be reduced by applying several pulses with short intervals between them. Thus, it is possible to stimulate an action potential with reduced stimulation intensity by increasing the duration of the stimulation. While increasing the duration of a single pulse might be painful or detrimental to the surface areas of the brain, summing a series of individual pulses over a duration of time could have the desired effect. Moreover, the combination of time and spatial summation may allow activation of neuronal pathways in deep brain regions, without activation of neurons in superficial regions. This is possible as the induced electric field in a deep region may be considerable during the period of pulses coming from different members of the coil, while the induced field in a superficial region closer to a given coil element is higher during a short time fraction of the total period of pulses, but lower during activation of the other coil elements. For example, the coil may be designed in a configuration such that the various members are scattered around a desired region or path, and may be stimulated consecutively so that at each time period only a certain element or group of elements is activated. This way, a significant electrical field can be induced at the desired region for all time periods, or with short inter-pulse intervals that may still enable activation, while in the cortical region of the brain, only certain regions will experience a significant field at certain periods, and the intervals between experiences of significant field will be much longer.

This effect can be accomplished by using more than one stimulator, or by using a configuration of a stimulator which includes multiple channels for stimulation. A multi-channel system may include multiple capacitors, each of which is discharged via an individual channel, with different switches into different coils or portions of the coil, or to different portions of the coil which are connected in parallel. A control unit can control the times of charging and discharging of each of the capacitors, and can also control delays between operation times of sequential coils.

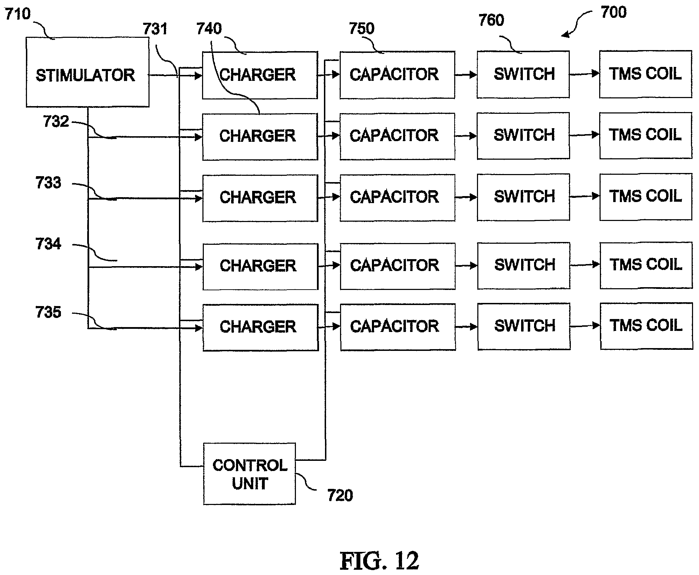

Reference is now made to FIG. 12, which is a block diagram illustration of a multi-channel TMS system 700, in accordance with embodiments of the present invention. System 700 includes a power supply 710, a control unit 720, and multiple channels 731-735. Although the example shown includes five individual channels, any suitable number of channels may be used. In some embodiments, two or more channels are used. In some embodiments, 3-5 channels are used. In other embodiments, maybe 10 channels may be used. Power supply 710 is connected to each of channels 731-735. Each channel 731-735 is further connected to a charger 740, one or more capacitors 750, a high current fast switch 760 through which capacitors 750 are discharged, and a selected portion of the TMS coil, or a separate coil. Control unit 720 is configured to control the charging voltage and timing, the discharge timing, delays between operation times of various coils or coil portions, and the current polarity in each channel, and may be digital, analog, or a combination thereof. Control unit 720 may be comprised of a processor embedded within system 700, or attached thereto. For example, an external PC may be connected to stimulator 710 and to each of channels 731-735. In the embodiment shown in FIG. 12, each capacitor is discharged through a separate switch to individual coils or portions of the coil.

Power supply 710 may include any kind of system which enables charging of capacitors to a high voltage. It may include a circuit which converts AC to DC, and amplifies the voltage. It may include an AC to DC transformer, IGBT transistor, FLYBACK system, Buck boost circuit, or any other system which can charge capacitors to high voltage. Power supply 710 may enable controlled charging of the capacitors. In some embodiments, system 700 may further include accumulators and/or batteries which may supply the energy for charging the capacitors. System 700 may include any combination of part or all of the mentioned components, and may be able to operate on one electric phase, three phases or both, on 220-230 V electricity mains, 110-115 V electricity mains, or any other electricity mains. System 700 may be specific to a certain type of electricity mains, or versatile and able to operate on two or more types of electric nets.

The maximal voltage on capacitor 750 may be a few hundred volts or more. Specific systems may be able to charge one or more capacitors 750 to voltages of 500 V, 1000 V, 1200 V, 1500, V, 1800 V, 2000 V, 3000 V. 5000 V or more.

Capacitors 750 may have capacitances from a few $\mu$f to hundreds of $\mu$f. In some embodiments, capacitors 750 may have capacitances of 40 to 60 $\mu$f. In some embodiments, capacitors 750 may have capacitances of 10 to 100 $\mu$f. In other embodiments, capacitors 750 may have capacitances of 10 to 300 $\mu$f. In other embodiments, capacitors 750 may have capacitances of 1 to 1000 $\mu$f or more. In some embodiments, capacitances in each of channels 731-735 are the same. In other embodiments some or all of the channels 731-735 may have different capacitances. In each channel there may be a single capacitor 750, or an array of two or more capacitors 750 connected in parallel, in series or in any combination of parallel and series.

Switch 760 may be a thyristor, a silicon controlled rectifier (SCR), an IGBT transistor, may include transistors, diodes, or any combination of both, based on MOSFET technology, TTL technology or any combination of both, MOS-gated thyristor, MOS controlled thyristor (MCT), or any switch that can pass high current in a short amount of time. Switch 760 should be able to pass electric current at a rate of a few tens of amperes per microsecond or higher. In some embodiments, switch 760 may be able to pass currents of up to 100 A/ps. In other embodiments, switch 760 may be able to pass currents of up to 120 A/$\mu$s, 150 A/ps, 200 A/$\mu$s, 500 A/$\mu$s or more. The peak current may be 100 Amperes or more. In some embodiments, switch 760 may be able to pass peak currents of 200 Amperes, 500 Amperes, 1000 Amperes, 2000 Amperes, 3000 Amperes, 5000 Amperes, 10000 Amperes or more. The pulse shape may be monophasic, biphasic, polyphasic, or other. In principle two or more switches may be connected to the same coil or element of coil. In some embodiments one switch is connected to each coil or element of coil. In some embodiments, a coil or element of coil may be connected to more than one switch, and may be activated in several periods by several channels.

In one embodiment, multiple individual stimulators may be used instead of a single stimulator including multiple channels, with an overall control unit for controlling of the timing of operation of each stimulator. In yet another embodiment, a combination of multiple individual stimulators, and multi-channel stimulators may be used.

In all of the above embodiments, various coils or portions of coil may be operated sequentially, with delay times in the microseconds, tens of microseconds, or hundreds of microseconds range. In each operation one TMS cycle is induced through a specific coil or portion of a coil. The cycle may be biphasic or monophasic, and the number of different coils or coil elements may vary. In some embodiments, three to five coils or portions of coil are operated consecutively. In other embodiments, 10 or more coils or portions of coil are stimulated. In other embodiments, two coils or portions of coil are operated consecutively. In some cases, some of the coils or portions of coil may be operated simultaneously in a certain period. In some cases, each coil or portion of coil is stimulated once, and in other embodiments, each coil or portion of coil is stimulated multiple times. Delay times may be the same for each stimulation, or may vary. Coils may be positioned at various regions on the subject's head, scattered around the deep brain region or several deep brain regions targeted for stimulation.

The following parameters are controllable by the operator of system 700: delay times between stimulation of different coils or portions of coil, number of channels and coils/coll portions stimulated, number of times each coil/coil portion is activated, timing of each activation, polarity of current in the coil, frequency of operation of each coil (i.e. number of pulses per second in each coil/coil portion), train duration of each coil, number of trains and inter-train intervals, and power output of operation for each coil. All these parameters may either be constant for different operations of a coil/coil portion, or may vary for different operations.

EXAMPLES

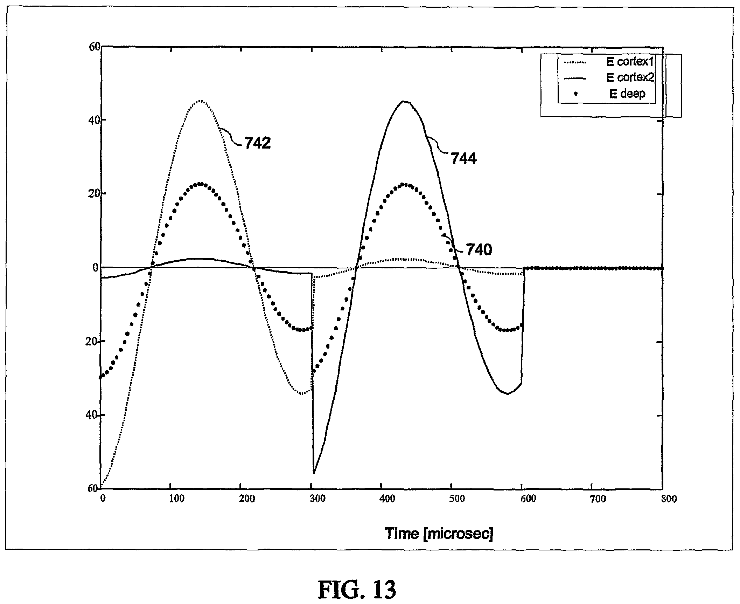

Reference is now made to FIG. 13, which is a graphical illustration of electric field pulses induced in a deep brain region (curve 740), in a first cortical region (curve 742) and a second cortical region (curve 744). The pulse of the second cortical region coil portion (curve 744) lags behind the pulse of the first cortical region coil portion (curve 742) by a full cycle. In each coil portion, one pulse cycle is induced, and the switch is disconnected at the end of a cycle when the current is zero. In the example shown in FIG. 13, the field intensity in the deep brain region is 50% of the intensity induced in the cortical region close to the coil portion adjacent to the first cortical region, while the field intensity in this same cortical region during operation of the coil portion close to the second cortical region is 5%. Thus, in the deep brain region a significant electric field is induced during two consecutive pulses, while in each of the cortical regions a significant field is induced only during one cycle. Thus, by alternating areas of cortical stimulation, different cortical regions get varying amounts of field intensity, while the deep brain region receives a consistent field intensity for the various periods of stimulation.

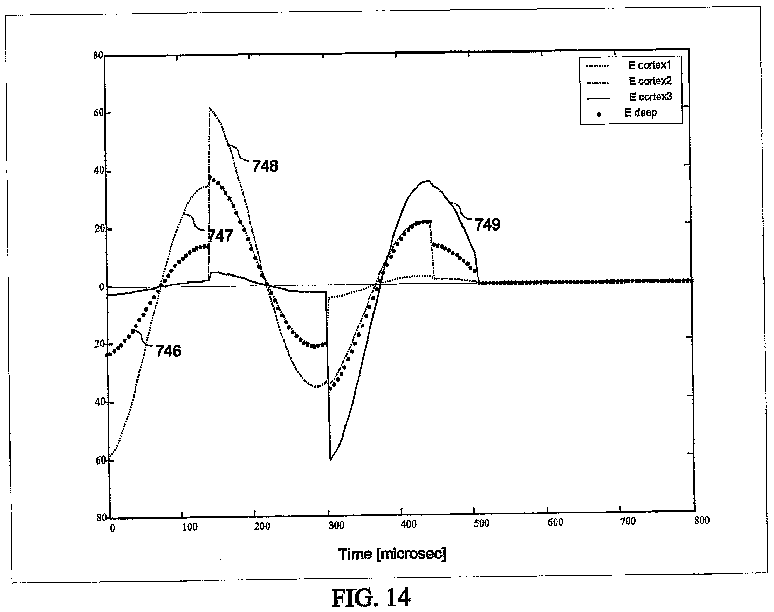

Reference is now made to FIG. 14, which is a graphical illustration of electric field pulses induced in a deep brain region (curve 746) and three cortical regions (curves 747, 748, and 749), where the time delay between two consecutive pulses is half a cycle, and the current polarity in the second pulse is opposite to the polarity in the first and third pulses. In each coil or coil portion, one pulse cycle is induced, and the switch is disconnected at the end of cycle when the current is zero. In this example, the field intensity in the deep brain region is 50% of the intensity induced in the cortical region close to the equivalent coil, while the field intensity in any one of the cortical regions during operation of the portion of coil close to another cortical region is 5%. In this example there is an extension of the duration at which the deep brain region is exposed to significant field, and in addition there is an increase in intensity after each half cycle. The absolute value of the maximum at the beginning of each pulse is higher than the next maxima of the same pulse due to the decay factor [alpha]=R/2L (Eq. 10), thus at the beginning of the second pulse the relationship between the field in the deep region and the field in the first cortical region will be higher with higher circuit resistance R, and with lower coil inductance L.

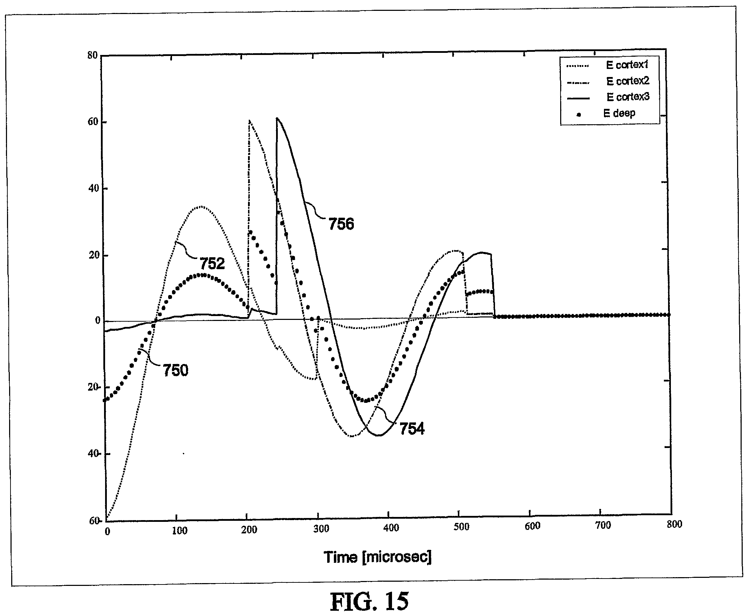

Reference is now made to FIG. 15, which is a graphical illustration of electric field pulses induced in a deep brain region (curve 750) and in three cortical regions (curves 752, 754, and 756), where the current polarities in the second and third pulses are opposite to the polarity in the first pulse, the time delay between the first and second pulse is approximately 3/4 cycle, and the time delay between the second and third pulse is about 1/8 of a cycle. In each coil one pulse cycle is induced, and the switch is disconnected at the end of a cycle when the current is zero. In this example, the field intensity in the deep brain region is 50% of the intensity induced in the cortical region close to the coil portion adjacent to the cortical region, while the field intensity in any one of the cortical regions during operation of the coil close to another cortical region is 5%. In this example there is extension of the duration of the positive half cycle in the deep brain region, and increase of the intensity.

It should be readily apparent that the same principles as described above with reference to FIGS. 13, 14 and 15 would apply to multiple coils or coil portions, and that similar methods could be employed using several or all of the coils or coil portions operating once or multiple times. Also, more than one pulse may be induced in part or all of the coils, thus increasing the time duration at which the deep brain region experiences significant induced fields. The overall stimulator output required to activate neuronal structures may thus be lowered, and the ability to activate deep brain regions with little or no activation of cortical regions may be improved.

A method of transcranial magnetic stimulation using temporal summation in accordance with one example of the disclosure is as follows. A coil 300 or 400 such as the one described above with reference to FIGS. 4 and 5 is placed on the skull. In one example, electrical leads are connected to one power supply or stimulator with multiple channels. In another example, additional leads are separately connected to two or more power supplies and to at least two members for providing electrical stimulation. Pulses are applied at a lower voltage and/or rate of change of electric current, so that the field induced at cortical brain regions will be sub-threshold or around the threshold level, but are applied to different members or groups of members at different times.

As one example, referring back to FIG. 5, elements B-C 401-407 may be connected to one channel, elements A-N 411-414 may be connected to another channel, and elements E-D 4Q1"-407" may be connected to different channels. The current polarity (direction and phase of current) may be the same or different in the separate channels. The windings connecting each element to the stimulator may be different from the windings shown in FIG. 5. The operator may control the timing of operation and current polarity in each channel. Each channel may be operated once or multiple times. For example one may operate elements B-C 401-407 for one pulse cycle, then elements E-D 401"-407" for one pulse cycle with a delay of full cycle, half a cycle, quarter of a cycle or any other delay time, followed by elements A-N 411-414 for one pulse cycle with a delay of full cycle, half a cycle, a quarter of a cycle or any other delay time, and again followed by elements B-C 401-407 for one pulse cycle with a delay of a full cycle, half a cycle, a quarter of a cycle or any other delay time, etc. In this way, one may achieve effective activation of deep brain region, with no or minimal activation of cortical regions. It should be noted that by using a multi-channel system, it is possible to control delay times and activation cycles, in order to fully optimize the strength/duration interplay for neuronal activation.

Various coils or elements of coil may be operated sequentially, with delay times of between zero and 1 ms or more, and specifically with delay times on the order of microseconds, tens of microseconds and/or hundreds of microseconds.

In one embodiment, different stimulations are applied at 100 microsecond intervals. In other embodiments the different pulses are applied at between 10 to 1000 microsecond intervals. In one embodiment, members are activated in a sequential order. In another embodiment, only certain members are activated, or a random pattern is generated. In other embodiments, groups of members are activated in a certain order.

In standard TMS stimulators, the operator may control the following parameters:

1. Power output, which determines the peak current passed through the coil and as a result the strength of the electric field induced.

2. Frequency, which determines the number of pulses passed through the coil each second. Accepted values may range from a single pulse up to 1 HZ, 10 Hz, 20 Hz, 30 Hz, 50 Hz and sometimes 100 Hz.

3. Train duration - The duration of a train of pulses. The duration and the frequency determine the number of pulses in a train. For example, in a train of 20 Hz with a duration of 2 seconds there are 40 pulses.

4. Inter-train interval - The intervals between consecutive trains. The intervals may be from a few seconds to a few minutes. The intervals may be constant over a session of trains, or variable along the session.

5. Number of trains in a session of trains.

In addition to all the above, in a multi-channel system such as the one disclosed herein, the operator may control the following parameters:

1. The timing of operation of each channel, and the time delays between consecutive channels. The time delays may be may be 1 microsecond, several microseconds, tens of microseconds, hundreds of microseconds, or more. The time delays may be the same between any consecutive channels, or there may be different time delays between different consecutive channels.
2. The current polarity in each channel, namely positive or negative current in the first stroke of the biphasic cycle, or the direction of a monophasic cycle.
3. The pulse shape in each channel, i.e. monophasic, biphasic, polyphasic, one cycle, two cycles, three cycles or more. In specific embodiments there may be one pulse cycle through each channel, as is customary in standard TMS stimulators.
4. The number of channels operated.
5. The order of operation of the channels. In some cases one or more channels may be operated more then once. For example a sequence of 10 operations may be programmed, using 3 channels, in the order: 1, 3, 2, 1, 3, 2, 1, 3, 2, 1. Another example of a sequence of 15 operations using 5 channels: 1, 5, 3, 4, 2, 1, 5, 3, 4, 2, 1, 5, 3, 4, 2. In some applications two or more channels may be connected to the same coil, so that this coil may be activated in several operations of different channels.
6. The power output, frequency, train duration, inter-train intervals, and number of trains for a session, in each channel. Part or all of the above parameters may be the same for all channels, or they may be different between part or all of the different channels.

In one embodiment, two or more of the various types of summation are combined. For example, morphological line spatial summation can be used at a sub-threshold intensity in combination with temporal summation. That is, different parts of an axonal bundle can be targeted selectively or sequentially, rather than simultaneously. Alternatively, one coil can include members for one-point summation and for morphological line spatial summation. Each of the member types can be simultaneously, sequentially or selectively stimulated.

A multi-channel TMS system such as the one described above may be used for activation of different body parts, i.e. different brain regions, with different power outputs, frequencies, pulse shapes, train durations, inter-train intervals, and/or number of trains for a session. The different regions may be activated either simultaneously or with any time delay between them. The time delays may be in the order of microseconds, tens of microseconds, hundreds of microseconds, milliseconds, or any combination thereof. Different stimulation protocols may induce inhibitory or excitatory activation. It may thus be possible to study numerous interactions between different neuronal structures in different brain regions.

In one example, a possible application of the multi-channel system may be activation of different body parts, i.e. different brain regions, with different frequencies. The different regions may be activated either simultaneously or with any time delay between them. As a non-limiting example, several studies found that high frequency repetitive TMS (rTMS) at 10 Hz or more directed to the left prefrontal cortex had anti-depressant effect in depression patients (Padberg F, Zwanzger P, Thoma H, Kathmann N, Haag C, Greenberg BD, Hampel H, Moller HJ. Repetitive transcranial magnetic stimulation (rTMS) in pharmacotherapy- refractory major depression: comparative study of fast, slow and sham rTMS. Psychiatry Res 1999; 88:163-171). It was also demonstrated that low frequency rTMS at 1 Hz directed to the right prefrontal cortex had an anti-depressant effect (Fitzgerald PB, Brown T, Marston NAU, Daskaladis ZJ1 Kulkarni J. A double-blind placebo controlled trial of transcranial magnetic stimulation in the treatment of depression. Arch Gen Psychiatry 2003; 60:1002-1008; Klein E, Kreinin I, Chistyakov A, Koren D, Mecz L, Marmur S, Ben-Shachar D, Feinsod M. Therapeutic efficacy of right pre-frontal slow repetitive transcranial magnetic stimulation in major depression: a double-blind controlled study. Arch Gen Psychiatry 1999; 56:315-320; Fitzgerald PB, Benitez J, De Castella A, Daskalakis ZJ, Brown TL, Kulkarni J. A randomized controlled trial of sequential bilateral repetitive transcranial magnetic stimulation for treatment-resistant depression. Am J Psychiatry 2006; 163:88-94). Using a multi-channel TMS system, it may be possible to activate left pre-frontal regions with high frequency rTMS, and right pre-frontal regions with low frequency rTMS, either simultaneously or with any time delay between them, thus possibly achieving enhanced therapeutic effect.

Another application may be connecting two, three or more capacitors in parallel (and/or in series) to one channel through one switch, thus controlling the pulse duration by changing the total circuit capacitance (Eq. 11). For example, one may connect three capacitors of 50 [mu]f in parallel, to obtain a total circuit capacitance of about 150 [mu]f, thus extending the pulse duration by about [Lambda]/3. Extending pulse duration by increasing the capacitance may provide savings in power consumption, energy, voltages and/or currents required for neuronal stimulation. The ability to use different values of capacitance may give additional investigational tools for researchers, such as comparing effects of varying the capacitance or the like.

The abilities of multi-channel TMS systems as described here to stimulate several brain regions either simultaneously or with any time delay between them, and to obtain more focal activation of deep brain regions relative to standard one channel TMS stimulators may be beneficial in a large variety of applications. The system and methods of the present invention described herein may be used for research purposes, i.e. by activating any deep brain region with any set of operational parameters. In addition, they may be used to study or treat a neurophysiological condition. A "neurophysiological condition" may be a pathological neurophysiological condition or a neurophysiological disorder, such as, but not limited to: clinical depression, non-clinical depression, dysthemia, bipolar disorder, drug addiction, substance abuse, anxiety disorder, obsessive compulsive disorder, schizophrenia, Parkinson's disease, Alzheimer's disease, post-traumatic stress disorder, addictions such as smoking and alcoholism, autism, eating disorders including obesity, bulimia and anorexia, and others.

EXAMPLE of TESTED COIL

Reference is now made to the following example, which together with the above descriptions, illustrate the invention in a non limiting fashion.

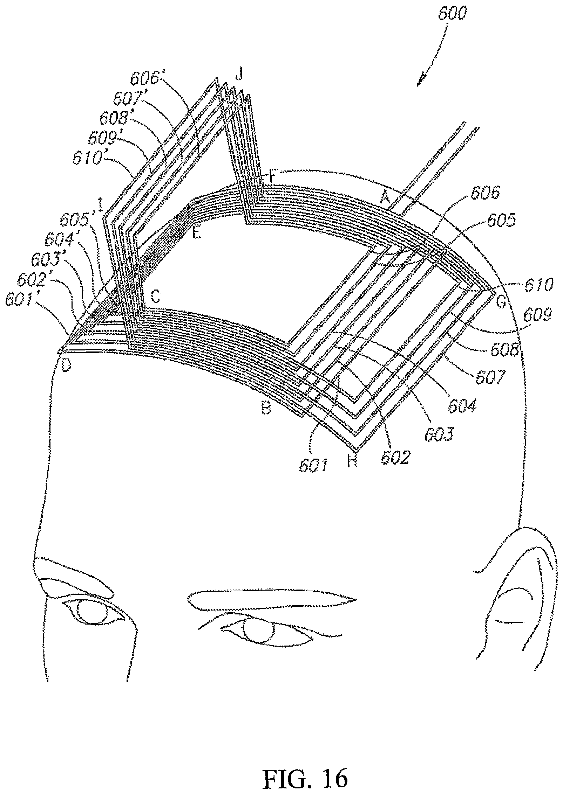

The biological efficacy of a coil similar to the ones described above with reference to FIGS. 4 and 5 was tested, using motor threshold as a measure of biological effect, as described in Zangen et al., "Transcranial magnetic stimulation of deep brain regions: evidence for efficacy of the H-coil", Clinical Neurophysiology 116:775-779, 2005. It should be noted that although the experimental coil, a schematic of which is shown in FIG. 16, is used to stimulate the motor cortex, a region which is accessible and measurable, the results can be appropriately compared to a coil designed to stimulate deep regions of the brain (which is more difficult to measure). This comparison was made possible by increasing the distance of the experimental coil from the site of activation (namely the motor cortex). Thus, a measure of the rate of decrease of electric field as a function of distance was taken at different distances from the skull. These measurements were compared to measurements taken under the same conditions using a standard figure-8 coil.

A coil 600 was designed to stimulate the right abductor pollicis brevis (APB), as shown in FIG. 16. The coil 600 has 10 members 601-610 split into two groups, designated by A-B and G-H in FIG. 16. The average length of the members is 11 cm. The only coil elements having radial current components are members 606-610, which are connected to the return paths shown in segments C- I and J-F. The length of the radial connecting elements is approximately 8 cm. The return paths of the other five members 601-605 are placed on the head at the contralateral hemisphere (segment D-E). The wires (segments B-C and F-A) connecting members 601-605 and return paths 601'-605" are approximately 9 cm long, on average. Coil 600 was compared to a standard commercial Magstim figure-8 coil with internal loop diameters of 7 cm.

Subjects were seated with the right forearm and hand supported. Motor evoked potentials of the right APB muscle were recorded using silver-silver chloride surface electrodes. Subjects were instructed to maintain muscle relaxation throughout the study. EMG amplitude was amplified using a conventional EMG machine (Counterpoint, Dantec Electronics, Skovlunde, Denmark) with band-pass between 10 and 2000 Hz. The signal was digitized at a frequency of 5 kHz and fed into a laboratory computer.

A Magstim Super Rapid stimulator (The Magstim Company New York, NY) which produces a bi-phasic pulse, coupled with either the figure-8 coil or the H-coil, was used. Preliminary studies showed the H-coil to have a loudness level of 122 dB when activated, similar to coils 300 and 400 described above in accordance with some examples of the present disclosure. Subjects were fitted with foam ear plugs to attenuate the sound.

Coil 600 was placed on the scalp over the left motor cortex. The intersection of the figure-8 coil was placed tangentially to the scalp with the handle pointing backward and laterally at a 45 degree angle away from the midline. Coils were held in a stable coil holder which could be adjusted at different heights above the "hot spot" on the scalp. Resting motor threshold was determined for each coil at different distances above the scalp, at increments of 0.5 cm.

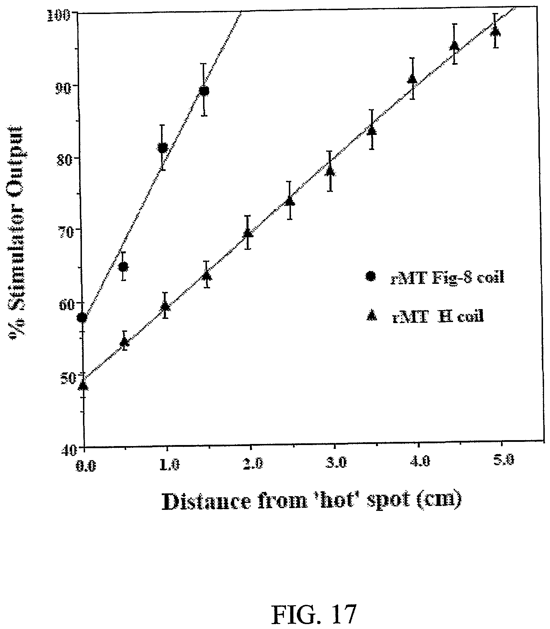

Reference is now made to FIG. 17, which is a graphical illustration of the results of the example described above. The graph shows the percentage of stimulator output needed to reach resting motor threshold as a function of distance of the coil from the "hot spot" on the skull for both coil 600 and the standard figure-8 coil. As shown in FIG. 17, the efficacy of coil 600 at large distances from the scalp was significantly greater than for the figure-8 coil. When using maximal stimulation power output, the figure-8 coil can be effective up to 2 cm away from the coil, while coil 600 can be effective at 5.5 cm away from the coil. Moreover, the rate of decay of effectiveness as a function of the distance from the coil is much slower in coil 600 relative to the figure-8 coil.

It should be readily apparent that the methods described herein may be applied to various types of TMS coils, and are not limited to the specific examples of TMS coils disclosed in the present application.

## Claims

1. A system (80, 700) for activating a neuronal structure, the system comprising:

   multiple stimulators or channels (731-735) for stimulating various coils sequentially, each of said multiple stimulators or channels having a capacitor and a switch, such that said system comprises multiple capacitors and multiple switches;
   a first transcranial magnetic stimulation (TMS) coil comprising a base portion having windings of multiple spaced apart members connected to one of said multiple stimulators or channels, and further connected to one of said multiple capacitors (750) and to one of said multiple switches (760);
   a second TMS coil comprising a base portion having windings of multiple spaced apart members connected to another of said multiple stimulators or channels and further connected to another of said multiple capacitors (750) and to another of said multiple switches (760);
   and
   a control unit (720) for controlling timing parameters for each of said multiple stimulators or channels, **characterised in that** said multiple stimulators or channels are configured to first activate said first TMS coil, and after a time interval of less than one millisecond to secondly activate said second TMS coil, wherein said first activation and said second activation are done using different stimulators or stimulator channels and wherein the different stimulator channels are stimulated consecutively such that at each time period at most one of the stimulator channels is active.

2. The system (80, 700) of claim 1, wherein the current pulse in some or all of the channels (731-735) may be monophasic or biphasic or polyphasic.

3. The system (80, 700) of claim 1, wherein said control unit (720) is configured to control at least some of: a charging voltage, charging timing, discharge timing, delays between operation of various coils or coil portions and current polarity.

4. The system of claim 1 (80, 700), wherein at least one of said multiple stimulators or channels (731-735) is capable of first activating said first TMS coil and after a first pre-determined period of time activating said second TMS coil.

5. The system (80, 700) of claim 4, wherein said first pre-determined period of time is within a range of 1-200 microseconds.

6. The system (80, 700) of claim 4, further comprising a third TMS coil and wherein at least one of said multiple stimulators (731-735) is capable of, after a second pre-determined period of time, activating a third TMS coil.

7. The system (80, 700) of claim 6, wherein said second pre-determined period of time is the same amount of time as said first pre-determined period of time.

8. The system (80, 700) of claim 1, wherein said first TMS coil and said second TMS coil are designed to carry current in predetermined directions.

9. The system (80, 700) of claim 8, wherein said predetermined directions are a same direction for each of said first and second TMS coils, and wherein the current polarity induced in said first and second TMS coils may be the same or opposite.

10. The system (80, 700) of claim 8, wherein said predetermined directions are a different direction for each of said first and second TMS coils, and wherein each of said predetermined directions forms a path designed to mimic a neuronal structure.

11. The system (80, 700) of claim 1, wherein said multiple stimulators or channels (731-735) are capable of selectively or repeatedly activating said first and second TMS coils.

12. The system (80, 700) of claim 1 wherein said multiple stimulators or channels (731-735) are capable of activating said first and second coils with same or different frequencies, train durations, inter-train intervals and/or number of trains.

13. The system of claim 1, wherein
the system is configured to repeatedly activate the first and second TMS coils.


**Patentansprüche**

1. System (80, 700) zum Aktivieren einer neuronalen Struktur, wobei das System umfasst:

mehrere Stimulatoren oder Kanäle (731-735) zum sequentiellen Stimulieren verschiedener Spulen, wobei jeder der mehreren Stimulatoren oder Kanäle einen Kondensator und einen Schalter aufweist, so dass das System mehrere Kondensatoren und mehrere Schalter umfasst;
eine erste Spule für die transkranielle Magnetstimulation (TMS), die einen Basisabschnitt umfasst, der Wicklungen aus mehreren voneinander beabstandeten Elementen aufweist, die mit einem der mehreren Stimulatoren oder Kanäle verbunden sind, und ferner mit einem der mehreren Kondensatoren (750) und einem der mehreren Schalter (760) verbunden ist;
eine zweite TMS-Spule, die einen Basisabschnitt umfasst, der Wicklungen aus mehreren voneinander beabstandeten Elementen aufweist, die mit einem anderen der mehreren Stimulatoren oder Kanäle verbunden sind und ferner mit einem anderen der mehreren Kondensatoren (750) und einem anderen der mehreren Schalter (760) verbunden sind; und
eine Steuereinheit (720) zum Steuern von Zeitsteuerungsparametern für jeden der mehreren Stimulatoren oder Kanäle, **dadurch gekennzeichnet, dass** die mehreren Stimulatoren oder Kanäle konfiguriert sind, um zuerst die erste TMS-Spule zu aktivieren und nach einem Zeitintervall von weniger als einer Millisekunde zum Zweiten die zweite TMS-Spule zu aktivieren, wobei die erste Aktivierung und die zweite Aktivierung unter Verwendung verschiedener Stimulatoren oder Stimulatorkanäle durchgeführt werden und wobei die verschiedenen Stimulatorkanäle nacheinander stimuliert werden, so dass zu jeder Zeitspanne höchstens einer der Stimulatorkanäle aktiv ist.

2. System (80, 700) nach Anspruch 1, wobei der Stromimpuls in einigen oder allen Kanälen (731-735) einphasig oder zweiphasig oder mehrphasig sein kann.

3. System (80, 700) nach Anspruch 1, wobei die Steuereinheit (720) konfiguriert ist, um mindestens einige von folgenden zu steuern: Ladespannung, Ladezeitpunkt, Entladezeitpunkt, Verzögerungen zwischen dem Betrieb verschiedener Spulen oder Spulenabschnitte und Strompolarität.

4. System nach Anspruch 1 (80, 700), wobei mindestens einer der mehreren Stimulatoren oder Kanäle (731-735) in der Lage ist, zuerst die erste TMS-Spule zu aktivieren und nach einer ersten vorbestimmten Zeitspanne die zweite TMS-Spule zu aktivieren.

5. System (80, 700) nach Anspruch 4, wobei die erste vorbestimmte Zeitspanne innerhalb eines Bereichs von 1 bis 200 Mikrosekunden liegt.

6. System (80, 700) nach Anspruch 4, ferner umfassend eine dritte TMS-Spule und wobei mindestens einer der mehreren Stimulatoren (731-735) nach einer zweiten vorbestimmten Zeitspanne in der Lage ist, eine dritte TMS-Spule zu aktivieren.

7. System (80, 700) nach Anspruch 6, wobei die zweite vorbestimmte Zeitspanne der gleiche Zeitraum ist wie die erste vorbestimmte Zeitspanne.

8. System (80, 700) nach Anspruch 1, wobei die erste TMS-Spule und die zweite TMS-Spule ausgelegt sind, um Strom in vorbestimmten Richtungen zu führen.

9. System (80, 700) nach Anspruch 8, wobei die vorbestimmten Richtungen für jede von der ersten und zweiten TMS-Spule dieselbe Richtung sind und wobei die in der ersten und zweiten TMS-Spule induzierte Strompolarität gleich oder entgegengesetzt sein kann.

10. System (80, 700) nach Anspruch 8, wobei die vorbestimmten Richtungen eine unterschiedliche Richtung für jede von der ersten und zweiten TMS-Spule sind und wobei jede der vorbestimmten Richtungen einen Pfad bildet, der ausgelegt ist, um eine neuronale Struktur nachzuahmen.

11. System (80, 700) nach Anspruch 1, wobei die mehreren Stimulatoren oder Kanäle (731-735) in der Lage sind, die erste und die zweite TMS-Spule selektiv oder wiederholt zu aktivieren.

12. System (80, 700) nach Anspruch 1, wobei die mehreren Stimulatoren oder Kanäle (731-735) in der Lage sind, die erste und die zweite Spule mit gleichen oder unterschiedlichen Frequenzen, Zugdauern, Intervallen zwischen Zügen und/oder Anzahl von Zügen zu aktivieren.

13. System nach Anspruch 1, wobei das System konfiguriert ist, um die erste und die zweite TMS-Spule wiederholt zu aktivieren.

**Revendications**

1. Système (80, 700) permettant d'activer une structure neuronale, le système comprenant :

de multiples stimulateurs ou canaux (731 à 735) pour stimuler diverses bobines séquentiellement, chacun desdits multiples stimulateurs ou canaux comportant un condensateur et un commutateur, de telle sorte que ledit système comprenne de multiples condensateurs et de multiples commutateurs ;
une première bobine de stimulation magnétique transcrânienne (TMS) comprenant une partie de base qui comporte des enroulements de multiples éléments espacés reliés à l'un desdits multiples stimulateurs ou canaux, et en outre reliés à l'un desdits multiples condensateurs (750) et à l'un desdits multiples commutateurs (760) ;
une deuxième bobine TMS comprenant une partie de base qui comporte des enroulements de multiples éléments espacés reliés à un autre desdits multiples stimulateurs ou canaux et en outre reliés à un autre desdits multiples condensateurs (750) et à un autre desdits multiples commutateurs (760) ; et
une unité de commande (720) pour commander des paramètres de synchronisation pour chacun desdits multiples stimulateurs ou canaux, **caractérisée en ce que** lesdits multiples stimulateurs ou canaux sont configurés pour activer d'abord ladite première bobine TMS, et après un laps de temps inférieur à une milliseconde pour activer ensuite ladite deuxième bobine TMS, ladite première activation et ladite seconde activation étant effectuées à l'aide de différents stimulateurs ou canaux de stimulation et lesdits différents canaux de stimulation étant stimulés consécutivement de telle sorte qu'à chaque période de temps, au plus l'un des canaux de stimulation soit actif.

2. Système (80, 700) selon la revendication 1, l'impulsion de courant dans certains ou tous les canaux (731 à 735) pouvant être monophasique ou biphasique ou polyphasique.

3. Système (80, 700) selon la revendication 1, ladite unité de commande (720) étant configurée pour commander au moins certains parmi : une tension de charge, une synchronisation de charge, une synchronisation de décharge, des retards entre le fonctionnement de diverses bobines ou parties de bobines et la polarité du courant.

4. Système selon la revendication 1 (80, 700), au moins l'un desdits multiples stimulateurs ou canaux (731 à 735) étant capable d'activer d'abord ladite première bobine TMS et après une première période de temps prédéfinie, d'activer ladite deuxième bobine TMS.

5. Système (80, 700) selon la revendication 4, ladite première période de temps prédéfinie se trouvant dans une plage de 1 à 200 microsecondes.

6. Système (80, 700) selon la revendication 4, comprenant en outre une troisième bobine TMS et au moins l'un desdits multiples stimulateurs (731 à 735) étant capable, après une seconde période de temps prédéfinie, d'activer une troisième bobine TMS.

7. Système (80, 700) selon la revendication 6, ladite seconde période de temps prédéfinie étant la même durée que ladite première période de temps prédéfinie.

8. Système (80, 700) selon la revendication 1, ladite première bobine TMS et ladite deuxième bobine TMS étant conçues pour transporter du courant dans des directions prédéfinies.

9. Système (80, 700) selon la revendication 8, lesdites directions prédéfinies étant une même direction pour chacune desdites première et deuxième bobines TMS, et ladite polarité du courant induite dans lesdites première et deuxième bobines TMS pouvant être la même ou opposée.

10. Système (80, 700) selon la revendication 8, lesdites directions prédéfinies étant une direction différente pour chacune desdites première et deuxième bobines TMS, et chacune desdites directions prédéfinies formant un chemin conçu pour imiter une structure neuronale.

11. Système (80, 700) selon la revendication 1, lesdits multiples stimulateurs ou canaux (731 à 735) étant capables d'activer de manière sélective ou répétée lesdites première et deuxième bobines TMS.

12. Système (80, 700) selon la revendication 1, lesdits multiples stimulateurs ou canaux (731 à 735) étant capables d'activer lesdites première et deuxième bobines avec des fréquences, des durées de trains, des intervalles inter-trains et/ou un nombre de trains identiques ou différents.

13. Système selon la revendication 1, ledit système étant configuré pour activer de manière répétée les première et deuxième bobines TMS.

FIG.1A

EP 1 890 762 B1

FIG.1B

FIG.2A

FIG.2B

FIG.3A

FIG. 3B

FIG.4

FIG.5

88

500    502    504    300,400

506

FIG.6

500

506    508    510    512

FIG.7

502

514    516

FIG.8

FIG.9

FIG. 10

FIG. 11

**FIG. 12**

EP 1 890 762 B1

FIG. 13

FIG. 14

FIG. 15

FIG. 16

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20050228209 A
- WO 0232504 A
- US 5738625 A
- GB 2196853 A
- JP 408280820 B
- EP 0692993 A

### Non-patent literature cited in the description

- **BOURLAND JD ; NYENHUIS JA ; NOE WA ; SCHAEFER JD ; FOSTER KS ; GEDDES LA.** *Proc. Int. Soc. Magnetic Resonance in Medicine 4th Scientific Meeting,* 1996, 1724
- **PADBERG F ; ZWANZGER P ; THOMA H ; KATHMANN N ; HAAG C ; GREENBERG BD ; HAMPEL H ; MOLLER HJ.** Repetitive transcranial magnetic stimulation (rTMS) in pharmacotherapy- refractory major depression: comparative study of fast, slow and sham rTMS. *Psychiatry Res,* 1999, vol. 88, 163-171
- **FITZGERALD PB ; BROWN T ; MARSTON NAU ; DASKALADIS ZJ1 ; KULKARNI J.** A double-blind placebo controlled trial of transcranial magnetic stimulation in the treatment of depression. *Arch Gen Psychiatry,* 2003, vol. 60, 1002-1008
- **KLEIN E ; KREININ I ; CHISTYAKOV A ; KOREN D ; MECZ L ; MARMUR S ; BEN-SHACHAR D ; FEINSOD M.** Therapeutic efficacy of right pre-frontal slow repetitive transcranial magnetic stimulation in major depression: a double-blind controlled study. *Arch Gen Psychiatry,* 1999, vol. 56, 315-320
- **FITZGERALD PB ; BENITEZ J ; DE CASTELLA A ; DASKALAKIS ZJ ; BROWN TL ; KULKARNI J.** A randomized controlled trial of sequential bilateral repetitive transcranial magnetic stimulation for treatment-resistant depression. *Am J Psychiatry,* 2006, vol. 163, 88-94
- **ZANGEN et al.** Transcranial magnetic stimulation of deep brain regions: evidence for efficacy of the H-coil. *Clinical Neurophysiology,* 2005, vol. 116, 775-779